# EUROPEAN PATENT SPECIFICATION

(11) **EP 2 882 330 B1**
(45) Date of publication and mention of the grant of the patent: **13.05.2020**
(21) Application number: 12837947.6
(22) Date of filing: 09.10.2012
(51) Int. Cl.: A61B 1/313, A61B 90/50, A61B 34/37, B25J 9/00

(54) **ROBOTIC SURGICAL DEVICES AND SYSTEMS**
ROBOTISCHE CHIRURGISCHE VORRICHTUNGEN UND SYSTEME
DISPOSITIFS CHIRURGICAUX ROBOTIQUES ET SYSTÈMES

(30) Priority: 08.08.2012 US 201261680809 P
(43) Date of publication of application: 17.06.2015
(60) Divisional application: 20166623.7
(73) Proprietor: Board of Regents of the University of Nebraska, Lincoln, NE 68583 (US)
(72) Inventor: FARRITOR, Shane, Lincoln, NE 68526 (US); MUMM, Erik, Longmont, CO 80504 (US); CHU, Philip, Friendswood, TX 77546 (US); KUMAR, Nishant, Bergenfield, NJ 07621 (US); DUMPERT, Jason, Omaha, NE 68135 (US); TSUTANO, Yutaka, Campbell, California 95008 (US)
(74) Representative: Boult Wade Tennant LLP
(86) International application number: PCT/US2012/000506
(87) International publication number: WO 2013/052137

(56) References cited:
- WO-A1-2011/075693
- WO-A1-2011/135503
- WO-A1-2011/135503
- US-A1- 2005 096 502
- US-A1- 2008 111 513
- US-A1- 2011 077 478
- US-A1- 2011 152 615
- US-A1- 2011 152 615
- US-A1- 2012 116 362
- US-A1- 2012 179 168

## Description

### TECHNICAL FIELD

The embodiments disclosed herein relate to various robotic devices that are disposed within a body cavity and positioned using a support component disposed through an orifice or opening in the body cavity.

### BACKGROUND

Invasive surgical procedures are essential for addressing various medical conditions. When possible, minimally invasive procedures such as laparoscopy are preferred.

However, known minimally invasive technologies such as laparoscopy are limited in scope and complexity due in part to 1) mobility restrictions resulting from using rigid tools inserted through access ports, and 2) limited visual feedback. Known robotic systems such as the *da Vinci*® Surgical System (available from Intuitive Surgical, Inc., located in Sunnyvale, CA) are also restricted by the access ports, as well as having the additional disadvantages of being very large, very expensive, unavailable in most hospitals, and having limited sensory and mobility capabilities.

There is a need in the art for improved surgical methods, systems, and devices.

WO 2011/135503 A1 discloses a robotic arm especially suited for laparoscopic surgery.
US 2011/152615 A1 discloses a surgical manipulator including an intracorporeal unit.
US 2012/0179168 A1 discloses various medical device components including components that can be incorporated into robotic and/or in vivo medical devices.
WO 2011/075693 A1 discloses modular medical devices.

### BRIEF DESCRIPTION OF THE DRAWINGS

FIG. 1A is a diagram showing a robotic surgical system, including a robotic device positioned inside a body, according to one embodiment.
FIG. 1B is a perspective view of the device of FIG. 1A.
FIG. 2A is a perspective view of a robotic medical device, according to one embodiment.
FIG. 2B is a perspective view of a robotic medical device showing the axes of rotation, according to one embodiment.
FIG. 3 is a perspective view of a robotic device and related equipment, according to one embodiment.
FIG. 4 is a perspective view of a robotic device and related equipment, according to one embodiment.
FIG. 5 is a perspective view of a robotic device and related equipment, according to one embodiment.
FIG. 6 is a perspective view of a robotic device poised to be inserted into a patient's cavity, according to one embodiment.
FIG. 7 is a side view of a robotic device during insertion and assembly, according to one embodiment.
FIG. 8 is another perspective view of the robotic device with an overtube for assembly, according to one embodiment.
FIG. 9 is another perspective view of the robotic device during assembly, according to one embodiment.
FIG. 10 is another perspective view of the robotic device and related equipment, according to one embodiment.
FIG. 11 is a view of a robotic device and related equipment, according to one embodiment.
FIG. 12A is a perspective view of a robotic medical device, according to one embodiment.
FIG. 12B is a cutaway perspective view of a robotic medical device, according to one embodiment.
FIG. 12C is a perspective view of a printed circuit board of a robotic medical device, according to one embodiment.
FIG. 12D is a cutaway perspective view of a robotic medical device, according to one embodiment.
FIG. 13A is a side cutaway view of a robotic medical device, according to one embodiment.

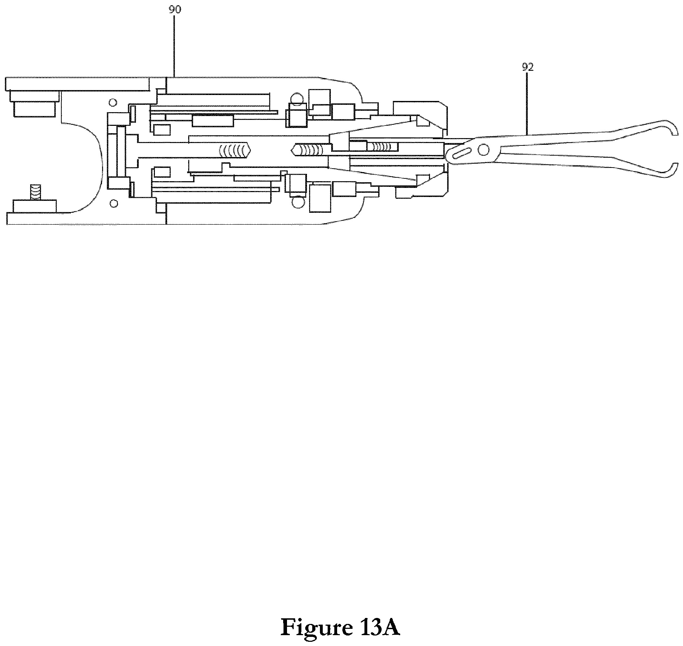

FIG. 13A is a side cutaway view of a robotic medical device, according to one embodiment.

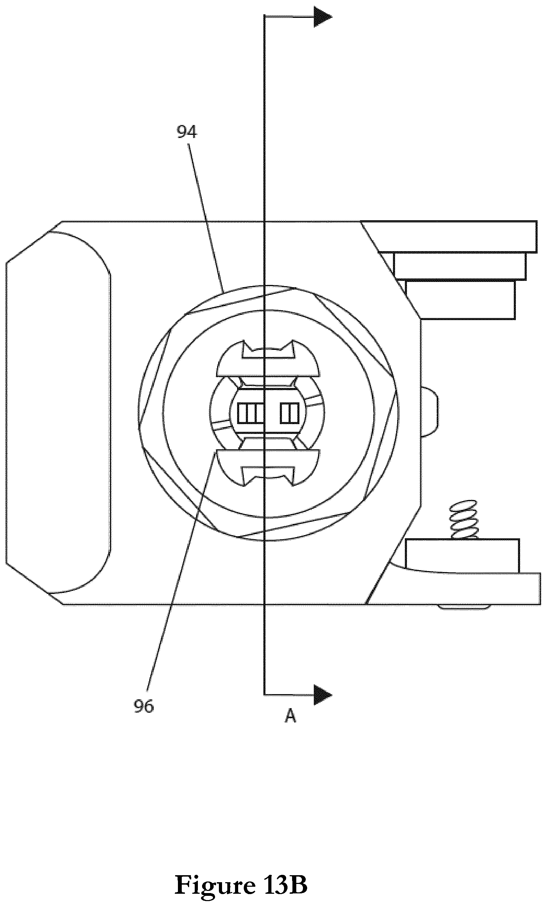

FIG. 13B is a front view of a forearm of a robotic medical device, according to one embodiment.

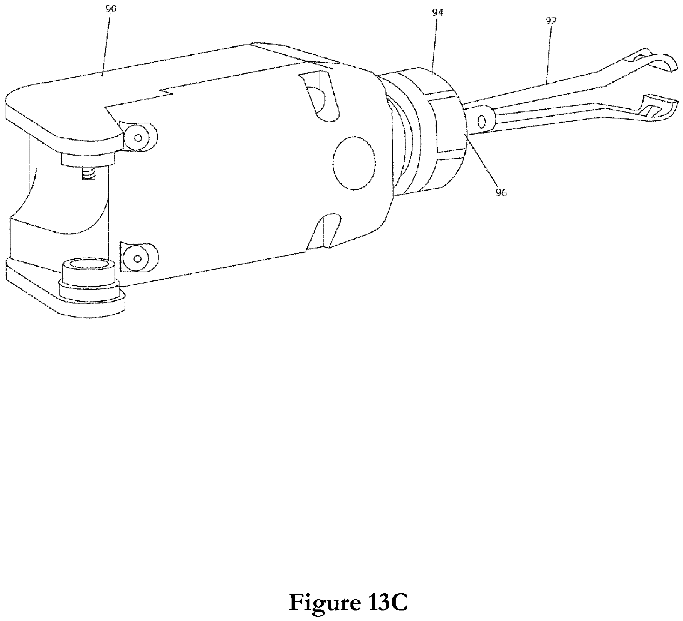

FIG. 13C is a rear perspective view of a forearm of a robotic medical device, according to one embodiment.

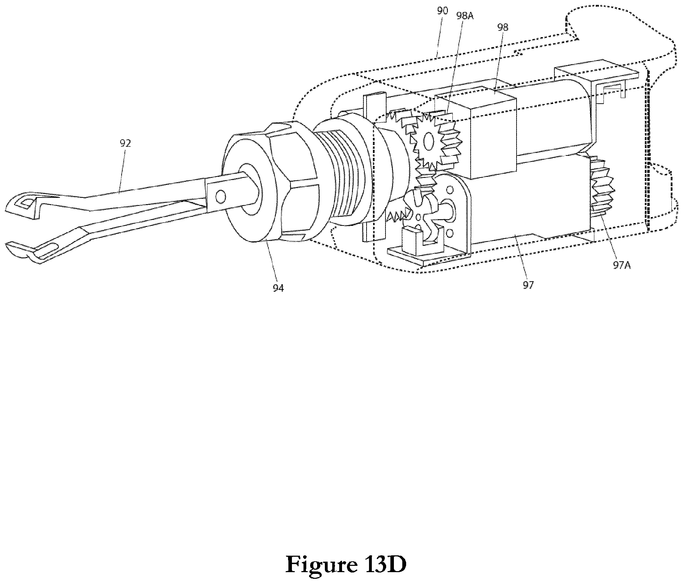

FIG. 13D is cutaway perspective view of a forearm of a robotic medical device, according to one embodiment.

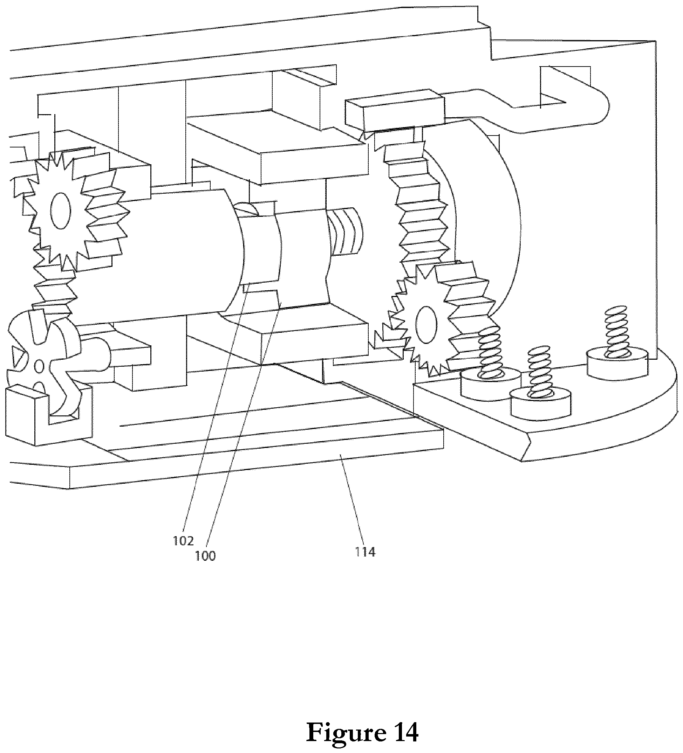

FIG 14 shows a cut away view of a robotic forearm, according to one embodiment.

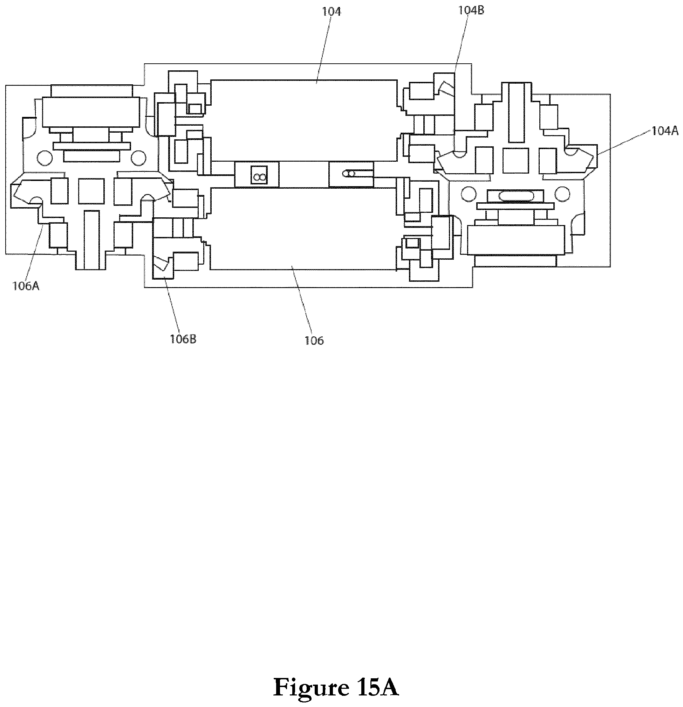

FIG 15A shows a cutaway side view of a robotic upper arm, according to one embodiment.

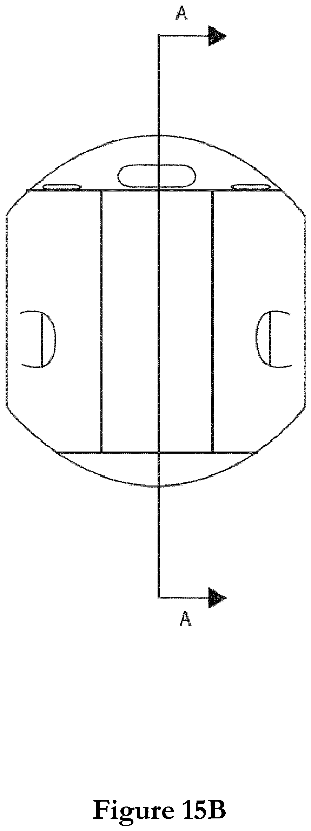

FIG 15B shows an end view of a robotic upper arm, according to one embodiment.

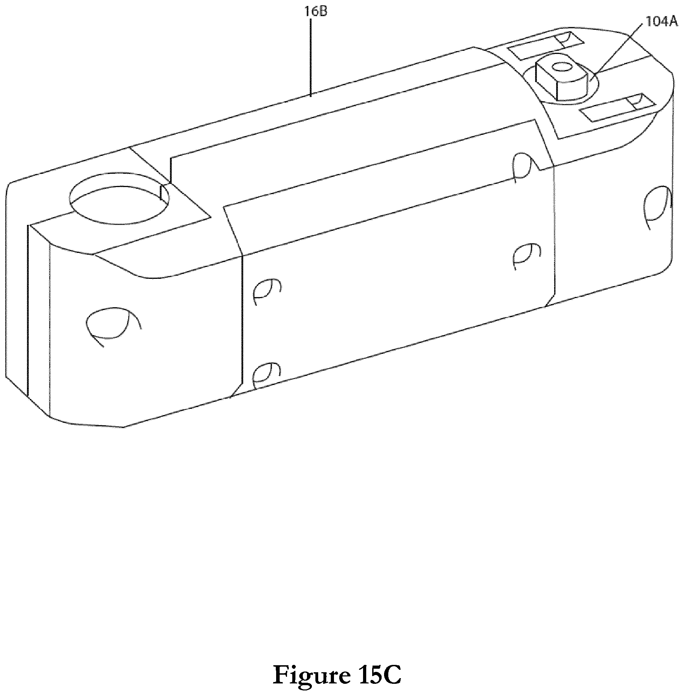

FIG 15C shows a perspective view of a robotic upper arm, according to one embodiment.

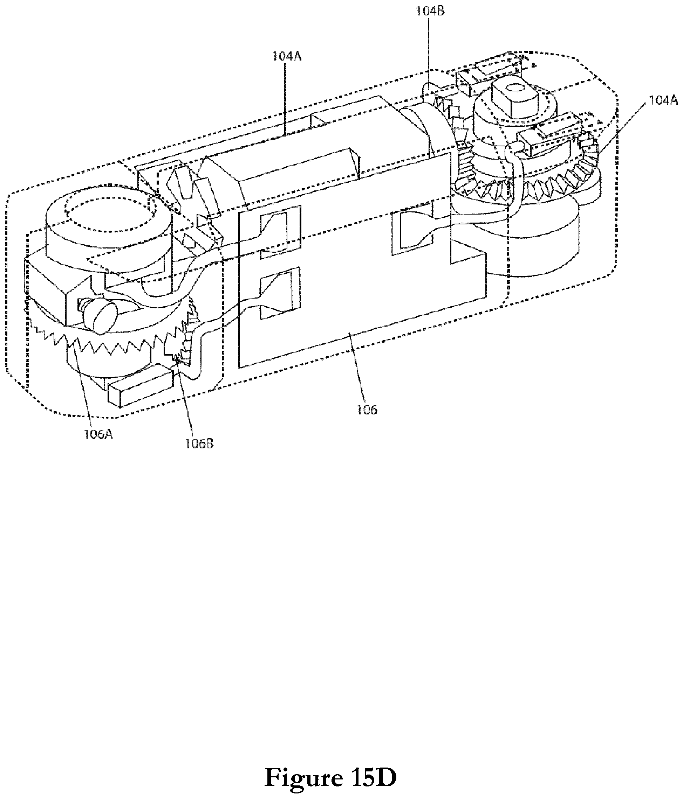

FIG 15D shows a cutaway perspective view of a robotic upper arm, according to one embodiment.

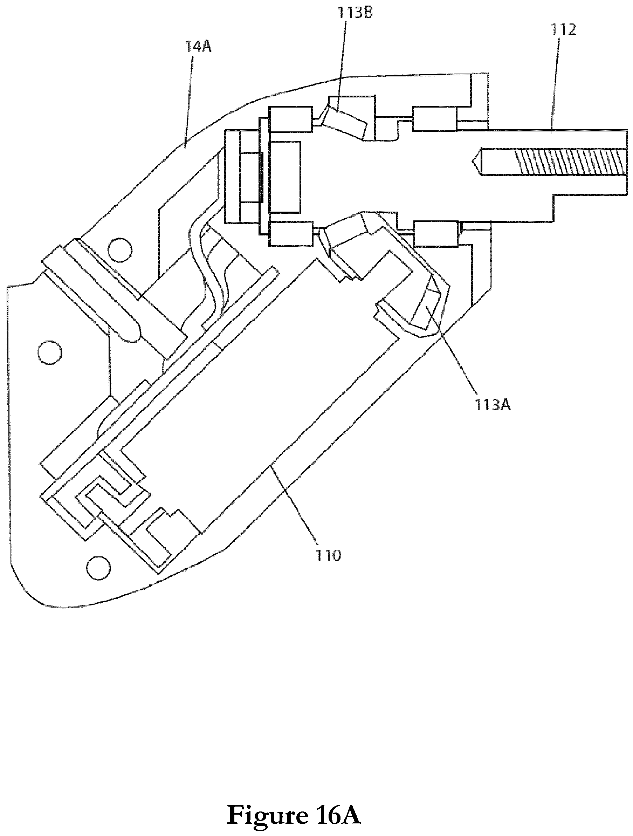

FIG 16A shows a cutaway side view of a robotic shoulder, according to one embodiment.

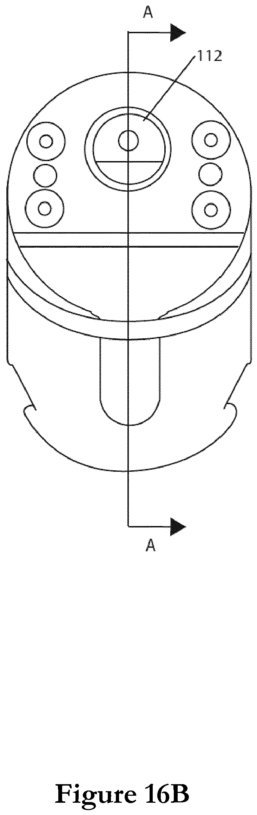

FIG 16B shows an end view of a robotic shoulder, according to one embodiment.

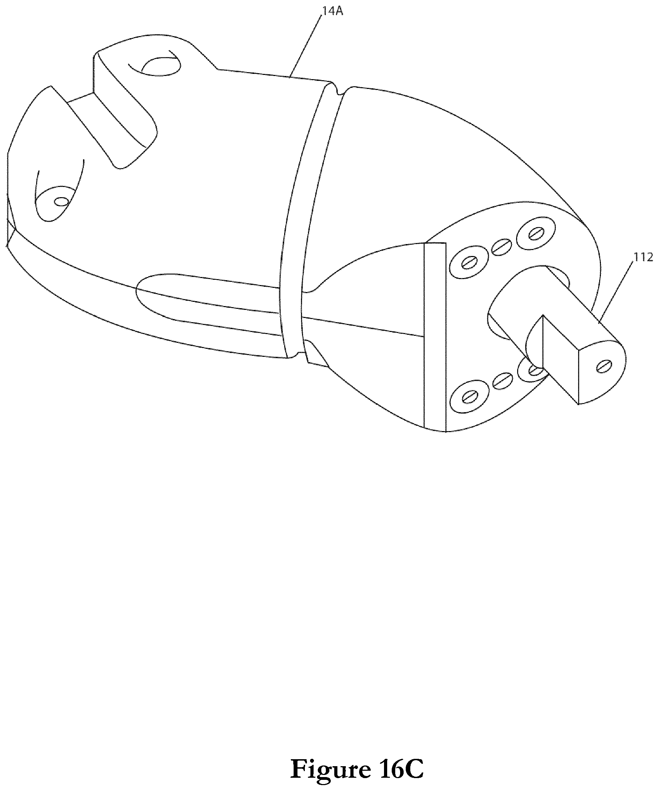

FIG 16C shows a perspective view of a robotic shoulder, according to one embodiment.

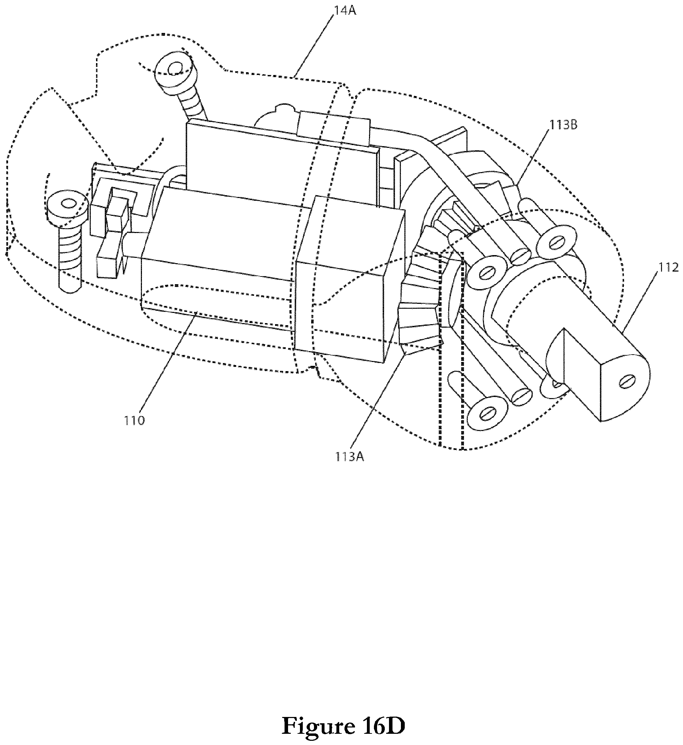

FIG 16D shows a perspective cutaway view of a robotic shoulder, according to one embodiment.

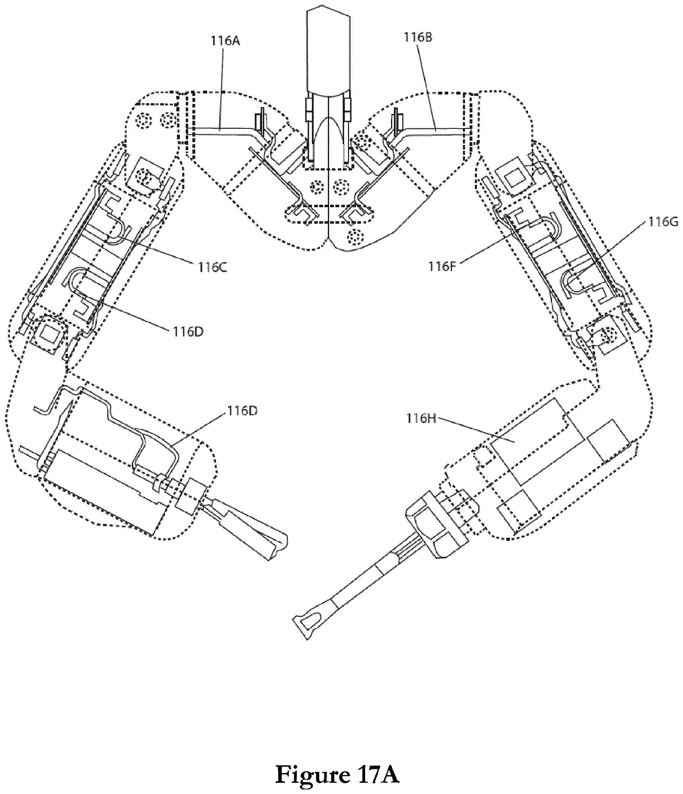

FIG 17A shows a top cutaway view of robotic device cabling, according to one embodiment.

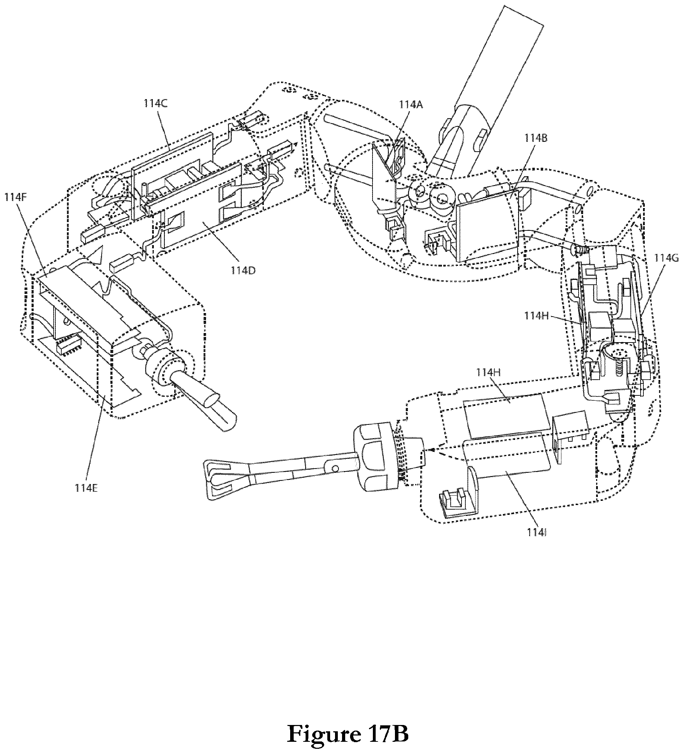

FIG 17B shows a cutaway perspective view of robotic device circuit boards, according to one embodiment.

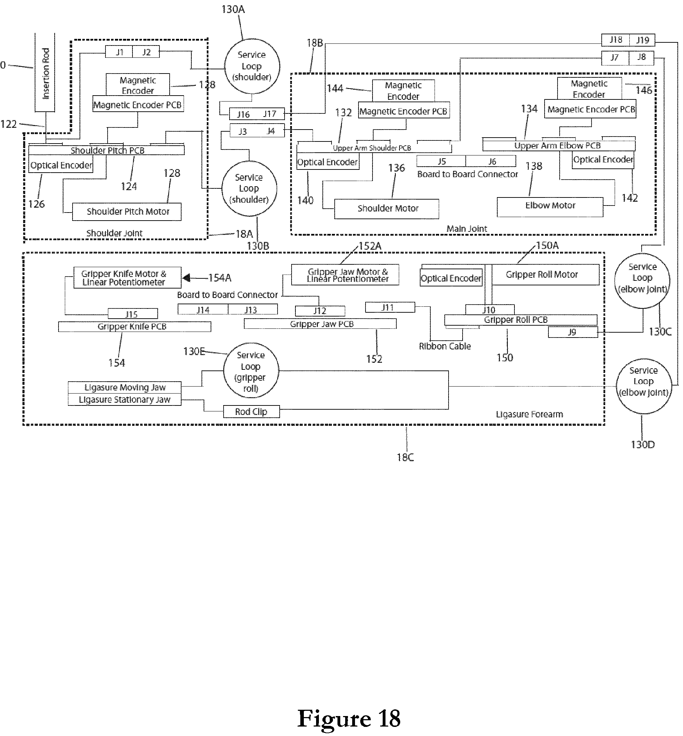

FIG 18 shows a block diagram of electronics for a robotic device/arm, according to one embodiment.

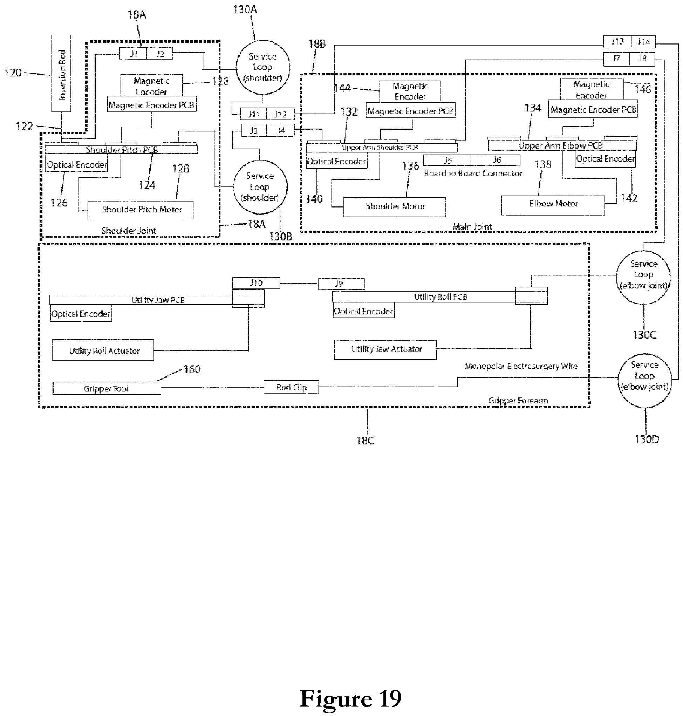

FIG 19 shows a block diagram of electronics for a robotic device/arm, according to one embodiment.

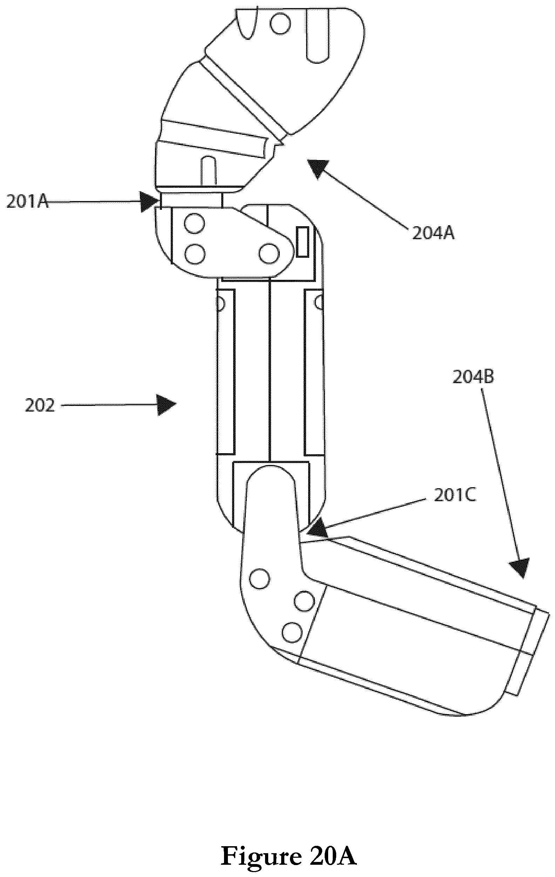

FIG 20A shows a robotic arm according to one embodiment.

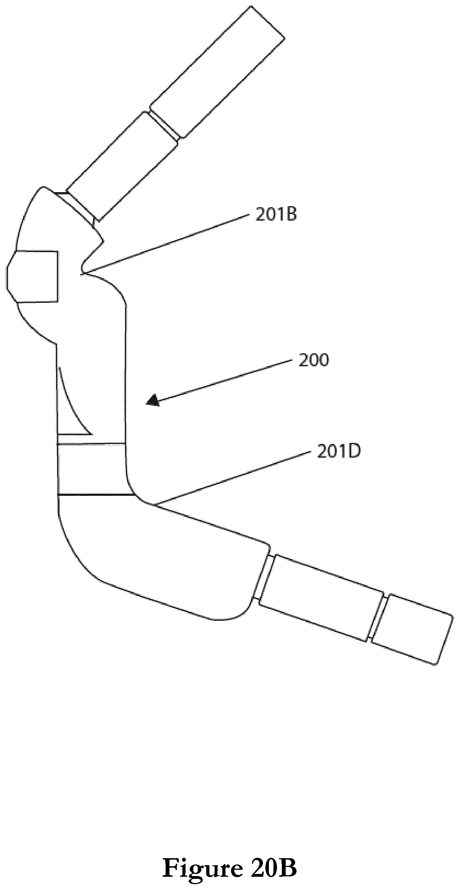

FIG 20B shows a robotic arm sleeve mold, according to one embodiment.

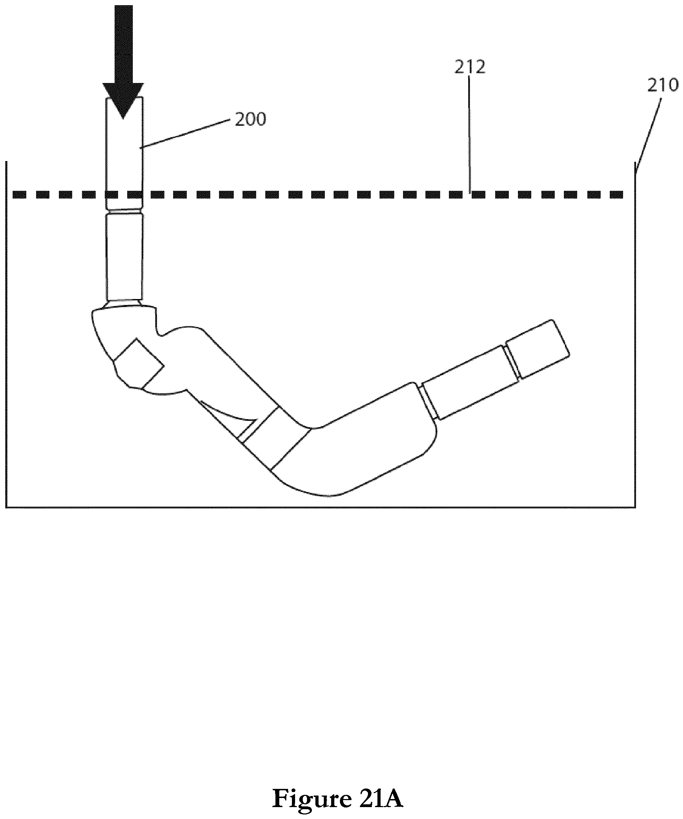

FIG 21A shows a robotic arm and sleeve making process overview, according to one embodiment.

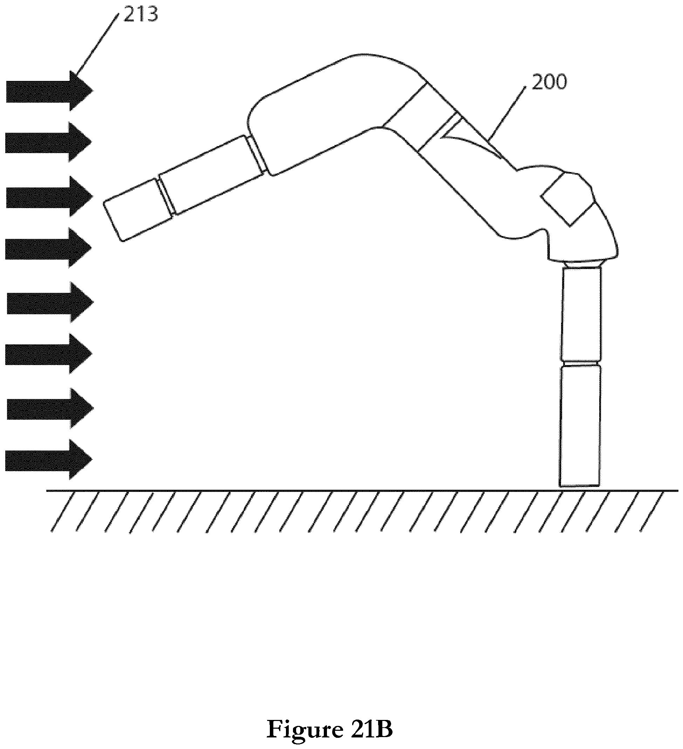

FIG 21B shows a robotic arm and sleeve making process overview, according to one embodiment.

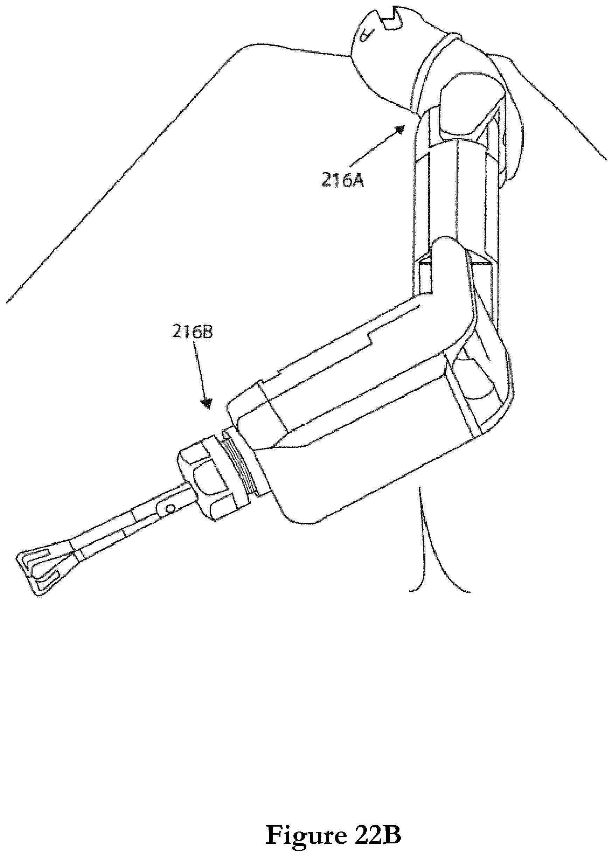

FIG 22B shows the rolled edges of the protective sleeve and the sleeve placed on the robotic arm, according to one embodiment.
FIG 22B shows the rolled edges of the protective sleeve and the sleeve placed on the robotic arm, according to one embodiment.

### DETAILED DESCRIPTION

The present invention is defined by the appended independent claim. Preferred embodiments are disclosed in the dependent claims. The various systems and devices disclosed herein include robotic devices and related systems.

The various embodiments disclosed herein may be incorporated into or used with any of the medical devices and systems disclosed in copending U.S. Applications 12/192,779 (filed on August 15, 2008 and entitled "Modular and Cooperative Medical Devices and Related Systems and Methods"), U.S. Patent 7,492,116 (filed on October 31, 2007 and entitled "Robot for Surgical Applications"), U.S. Patent 7,772,796 (filed on April 3, 2007 and entitled "Robot for Surgical Applications"), 11/947,097 (filed on November 27, 2007 and entitled "Robotic Devices with Agent Delivery Components and Related Methods), 11/932,516 (filed on October 31, 2007 and entitled "Robot for Surgical Applications"), 11/766,683 (filed on June 21, 2007 and entitled "Magnetically Coupleable Robotic Devices and Related Methods"), 11/766,720 (filed on June 21, 2007 and entitled "Magnetically Coupleable Surgical Robotic Devices and Related Methods"), 11/966,741 (filed on December 28, 2007 and entitled "Methods, Systems, and Devices for Surgical Visualization and Device Manipulation"), 12/171,413 (filed on July 11, 2008 and entitled "Methods and Systems of Actuation in Robotic Devices"), 60/956,032 (filed on August 15, 2007), 60/983,445 (filed on October 29, 2007), 60/990,062 (filed on November 26, 2007), 60/990,076 (filed on November 26, 2007), 60/990,086 (filed on November 26, 2007), 60/990,106 (filed on November 26, 2007), 60/990,470 (filed on November 27, 2007), 61/025,346 (filed on February 1, 2008), 61/030,588 (filed on February 22, 2008), 61/030,617 (filed on February 22, 2008), U.S. Patent 8,179,073 (issued May 15, 2011, and entitled "Robotic Devices with Agent Delivery Components and Related Methods"), 12/324,364 (filed 11/26/08, U.S. Published App. 2009/0171373 and entitled "Multifunctional Operational Component for Robotic Devices"), and 13/493,725 (filed 6/11/2012 and entitled "Methods, Systems, and Devices Relating to Surgical End Effectors").

Certain device and system implementations disclosed in the applications listed above can be positioned within a body cavity of a patient in combination with a support component similar to those disclosed herein. An "in vivo device" as used herein means any device that can be positioned, operated, or controlled at least in part by a user while being positioned within a body cavity of a patient, including any device that is coupled to a support component such as a rod or other such component that is disposed through an opening or orifice of the body cavity, also including any device positioned substantially against or adjacent to a wall of a body cavity of a patient, further including any such device that is internally actuated (having no external source of motive force), and additionally including any device that may be used laparoscopically or endoscopically during a surgical procedure. As used herein, the terms "robot," and "robotic device" shall refer to any device that can perform a task either automatically or in response to a command.

Certain embodiments provide for insertion of the present invention into the cavity while maintaining sufficient insufflation of the cavity. Further embodiments minimize the physical contact of the surgeon or surgical users with the present invention during the insertion process. Other implementations enhance the safety of the insertion process for the patient and the present invention. For example, some embodiments provide visualization of the present invention as it is being inserted into the patient's cavity to ensure that no damaging contact occurs between the system/device and the patient. In addition, certain embodiments allow for minimization of the incision size/length. Further implementations reduce the complexity of the access/insertion procedure and/or the steps required for the procedure. Other embodiments relate to devices that have minimal profiles, minimal size, or are generally minimal in function and appearance to enhance ease of handling and use.

Certain implementations disclosed herein relate to "combination" or "modular" medical devices that can be assembled in a variety of configurations. For purposes of this application, both "combination device" and "modular device" shall mean any medical device having modular or interchangeable components that can be arranged in a variety of different configurations. The modular components and combination devices disclosed herein also include segmented triangular or quadrangular-shaped combination devices. These devices, which are made up of modular components (also referred to herein as "segments") that are connected to create the triangular or quadrangular configuration, can provide leverage and/or stability during use while also providing for substantial payload space within the device that can be used for larger components or more operational components. As with the various combination devices disclosed and discussed above, according to one embodiment these triangular or quadrangular devices can be positioned inside the body cavity of a patient in the same fashion as those devices discussed and disclosed above.

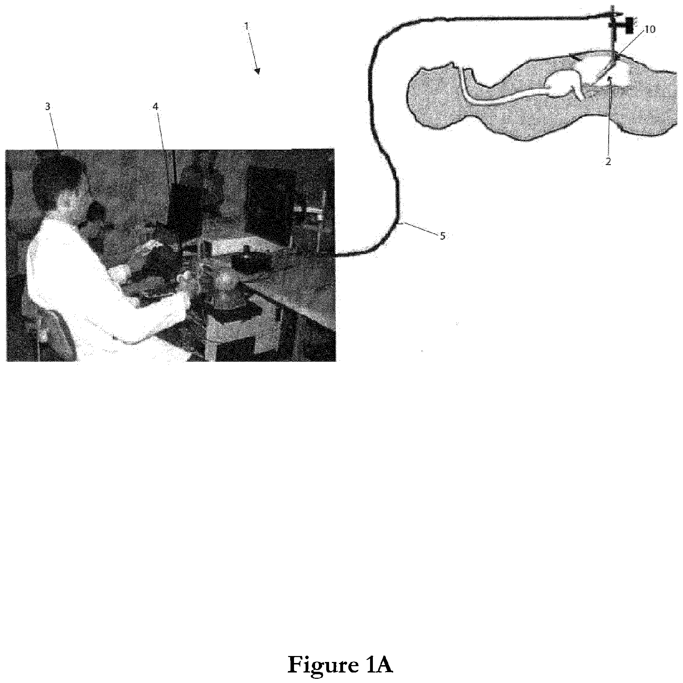

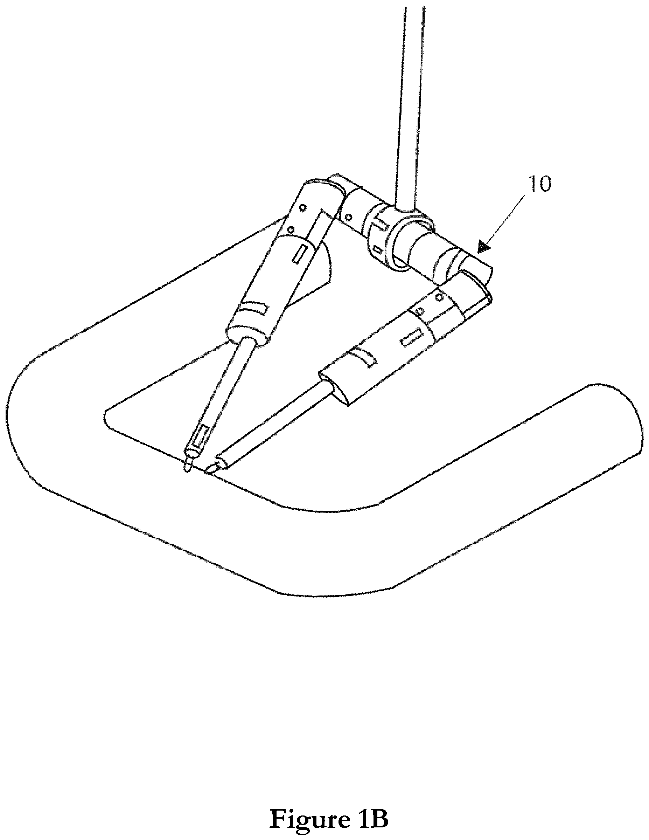

FIGS. 1A and 1B depict an exemplary system 1 that includes a robotic surgical device 10 disposed within the inflated peritoneal cavity 2 of a patient. It is understood that the various device and system embodiments disclosed herein, including the system 1 of FIGS. 1A and 1B, can be used for a variety of surgical procedures and tasks including, but not limited to, tissue biopsy, tissue dissection, or tissue retraction. For example, as shown in FIGS. 1A and 1B in accordance with one embodiment, the device 10 can be used to dissect tissue in the peritoneal cavity 2. In this system embodiment, a user (such as, for example, a surgeon) 3 operates a user interface 4 to control the device 10. The interface 4 is operably coupled to the device 10 by a cable 5 or other type of physical connection that provides for electronic power and/or electrical communication back and forth between the interface 4 and the device 10. Alternatively, the interface 4 can be operably coupled to the device 10 wirelessly. It is understood that the device embodiments disclosed herein can also be used with any other known system, including any of the systems disclosed in the various patent applications referred to herein.

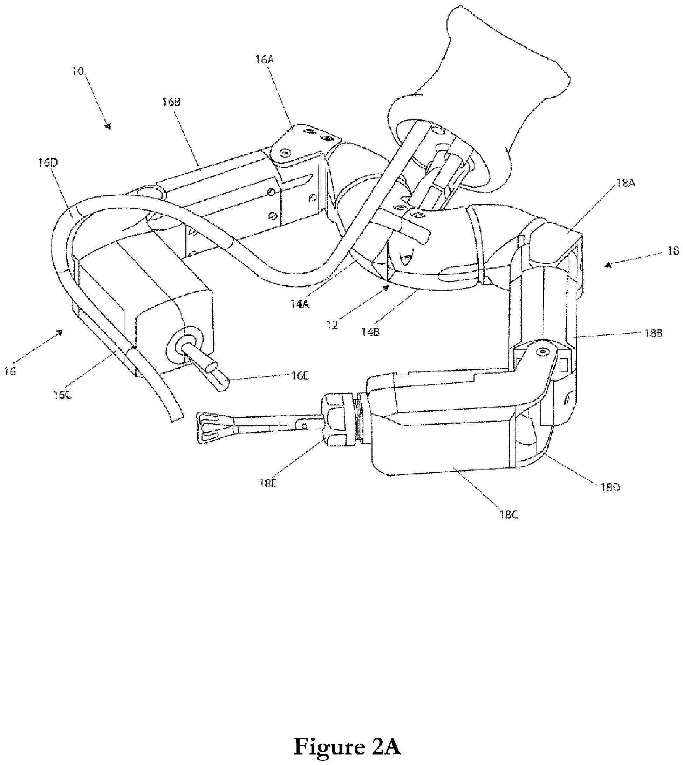

FIG. 2A depicts a robotic medical device 10, in accordance with one implementation. According to one embodiment, the device is an *in vivo* device. This device 10 embodiment as shown includes a body 12 that has two components 14A, 14B, which in this embodiment are cylindrical components 14A, 14B at an approximately 120 degree angle to each other. The cylindrical components 14A, 14B can also be referred to herein as shoulders, including a right shoulder 14A and a left shoulder 14B. In the embodiment depicted in FIG. 2A, the two components 14A, 14B are coupled directly to each other. Alternatively, the two components are not coupled to each other or, in another option, can be individually coupled to an access port used in the surgery.

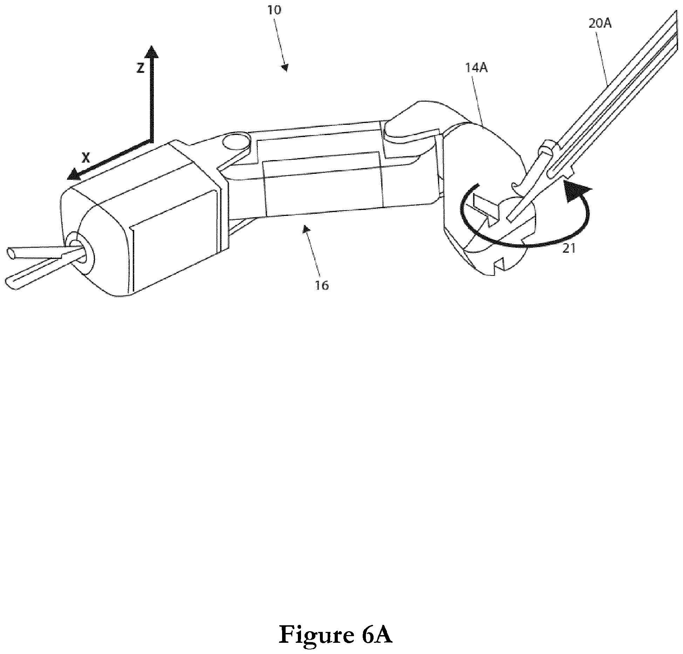

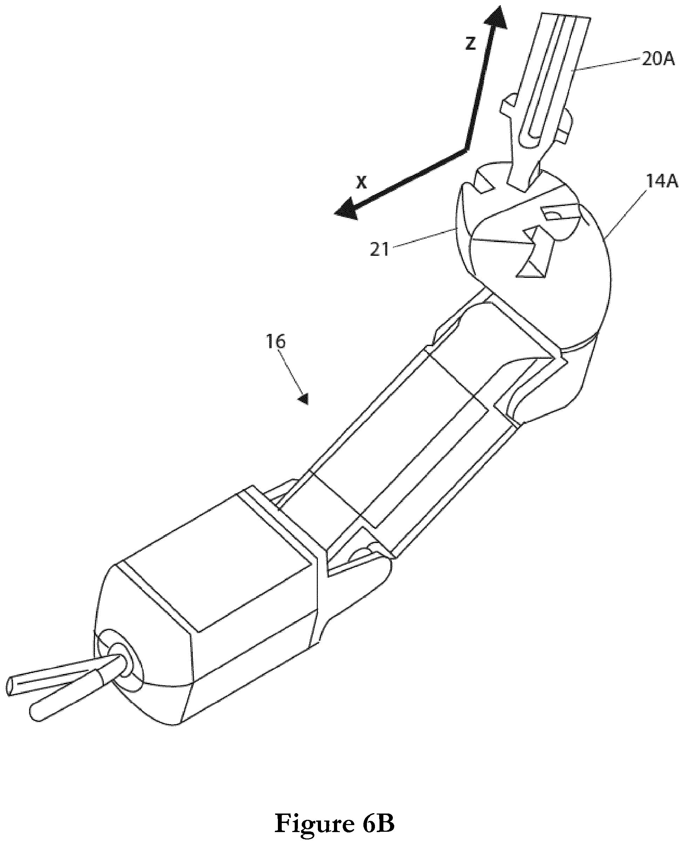

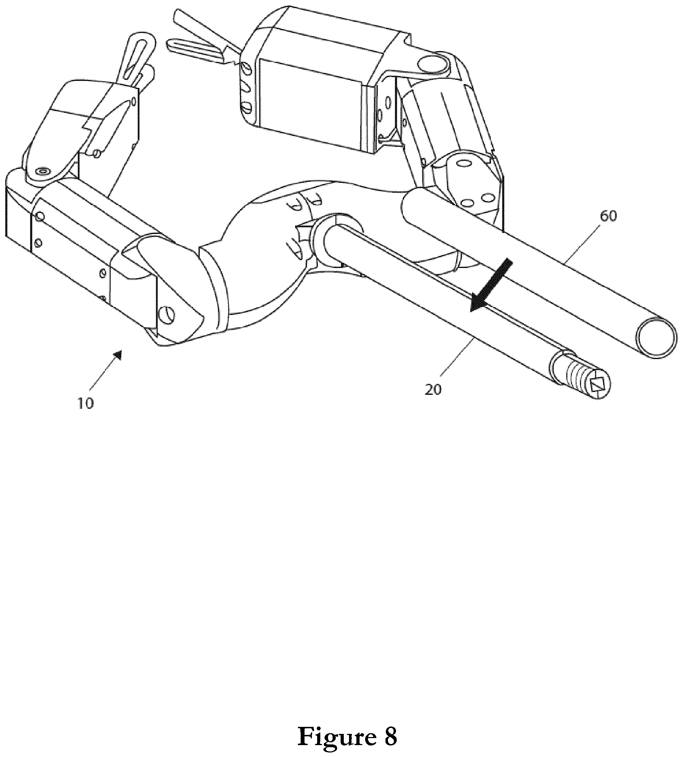

The body 12 is connected to two arms 16, 18 in one example of the device. In the implementation shown, the right shoulder 14A is coupled to right arm 16 and left shoulder 14B is coupled to left arm 18. In addition, the body 12 is also coupled to a support component 20, as best shown in FIG. 8. In accordance with one implementation as shown in FIGS. 6A and 6B and described in additional detail below, the support rod 20 as configured is a support rod 20 that is made of two coupleable support rod components 20A, 20B, each of which is independently attached to one of the body components 14A, 14B. More specifically, the support component 20 has a first support rod component 20A that is coupled to the first shoulder 14A and a second support rod component 20B that is coupled to the second shoulder component 14B. In certain implementations, the support component 20 can be a rod, tube, or other applicable shape.

Returning to FIG. 2A, each of the arms 16, 18 have a first joint 16A, 18A (each of which can also be referred to as a "shoulder joint") that is coupled to the body components 14A, 14B. Each first joint 16A, 18A is coupled to a first link 16B, 18B (also referred to as a "first segment," an "upper segment," or an "upper arm"), each of which is rotatably coupled to a second link 16C, 18C (also referred to as a "second segment," a "lower segment," or a "forearm") via a second joint 16D, 18D (each of which can also be referred to as an "elbow joint"). In addition, each arm 16, 18 also has an operational component (also referred to as an "end effector") 16E, 18E coupled to the forearm 16C, 18C. It is understood that the operational components 16E, 18E (and any of the operational components on any of the embodiments disclosed herein) can be any known operational components, including any of the operational components disclosed in the various patent applications referenced herein. By way of example, the components 16E, 18E can be cautery devices, suturing devices, grasping devices, imaging devices, operational arm devices, sensor devices, lighting devices or any other known types of devices or components for use in surgical procedures.

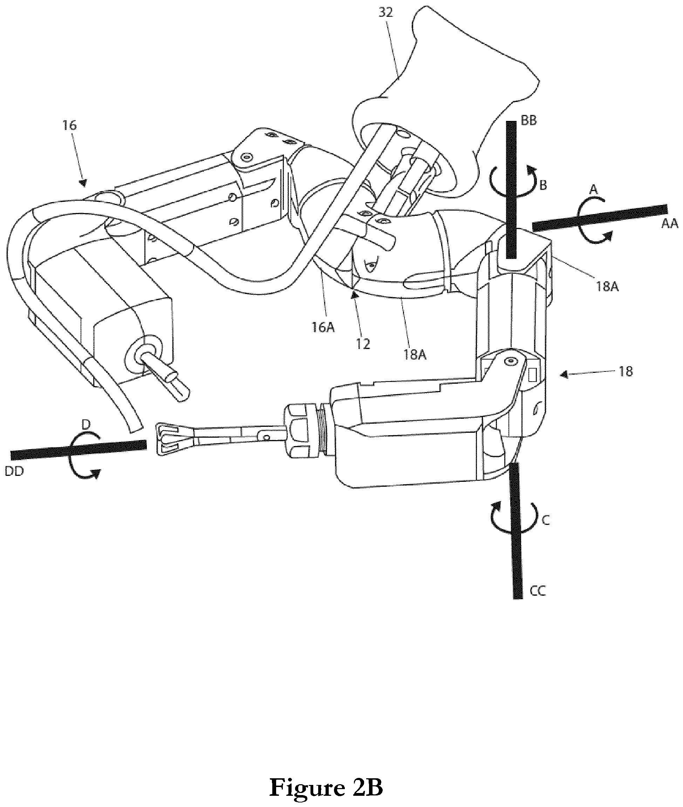

As mentioned above and as shown in FIG. 2B, the first links 16B, 18B are coupled to the body 12 via shoulder joints 16A, 18A. Each shoulder joint 16A, 16B is a joint having two axes of rotation. For example, as will be described in further detail below, the left shoulder joint 18A can be configured to result in rotation of the upper arm 18B as shown by arrow A around axis AA (that substantially corresponds to the longitudinal axis of the body 12) and also as shown by arrow B around axis BB, which is substantially perpendicular to axis AA. Because right shoulder joint 16A and right upper arm 16B are substantially the same as the left shoulder joint 18A and the left upper arm 18B, the above description also applies to those substantially similar (or identical) components. Alternatively, any known joint can be used to couple the upper arms 16B, 18B to the body 12.

Continuing with FIG. 2B, the upper arms 16B, 18B, according to one implementation, are coupled to the forearms 16C, 18C, respectively, at the elbow joints 16D, 16D such that each of the forearms 16C, 18C can rotate. For example, the forearms 16C, 18C can rotate as shown by arrow C around axis CC. Further, the end effectors 16E, 18E can also rotate relative to the forearms 16C, 18C, respectively, as shown by arrow D around axis DD. In addition, each of the operational components 16E, 18E can also be actuated to move between at least two configurations, such as an open configuration and a closed configuration. Alternatively, the operational components 16E, 18E can be coupled to the forearms 16C, 18C, respectively, such that the operational components 16E, 18E can be moved or actuated in any known fashion.

According to one embodiment, the operational components 16E, 18E, such as graspers or scissors, are also removable from the forearms 16C, 18C, such that the operational components 16E, 18E are interchangeable with other operational components configured to perform other/different types of procedures. Returning to FIG. 2A, one operational component 16E is a grasper 16E commonly known as a babcock grasper and the other 18E is a vessel sealing grasper 18E. Alternatively, either or both of the components 16E, 18E can be cautery devices, suturing devices, grasping devices, or any other known types of devices or components for use in surgical procedures, or can be easily replaced with such components.

It is understood that the device 10 in this embodiment contains the motors (also referred to as "actuators," and intended to include any known source of motive force) that provide the motive force required to move the arms 16, 18 and the operational components 16E, 18E. In other words, the motors are contained within the device 10 itself (either in the body, the upper arms, the forearms or any and all of these), rather than being located outside the patient's body. Various motors incorporated into various device embodiments will be described in further detail below.

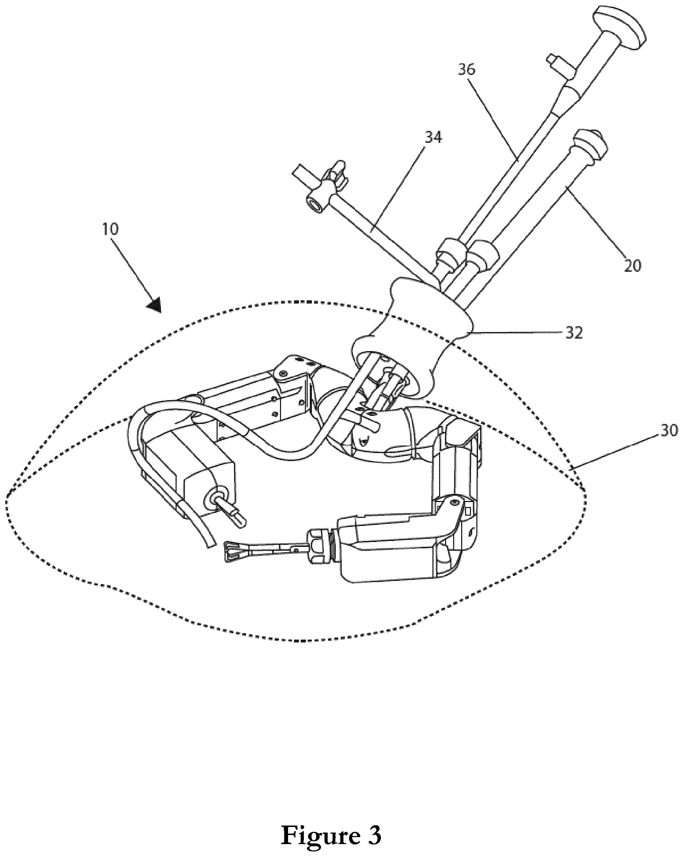

In use, as in the example shown in FIG. 3, the device 10 is positioned inside a patient's body cavity 30. For example, in FIG. 3, the body cavity 30 is the peritoneal cavity 30.

According to one implementation, the device 10 can be sealed inside the insufflated abdominal cavity 30 using a port 32 designed for single incision laparoscopic surgery. Alternatively, the device 10 can be inserted via a natural orifice, or be used in conjunction with other established methods for surgery. The device 10 is supported inside the abdominal cavity using the support rod 20 discussed above. The laparoscopic port 32 can also be used for insertion of an insufflation tube 34, a laparoscope 36 or other visualization device that may or may not be coupled to the device assembly. As an example, a 5 mm laparoscope 36 is shown in FIG. 3.

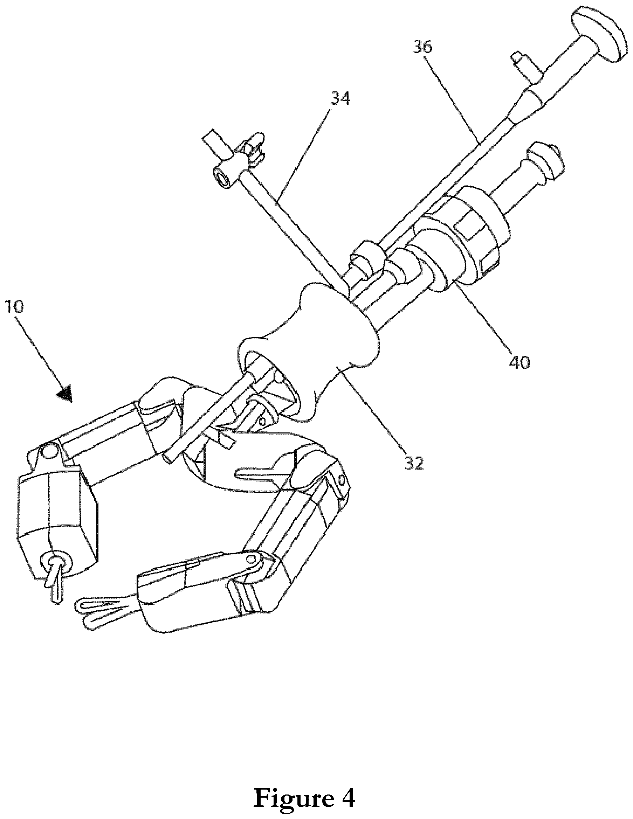

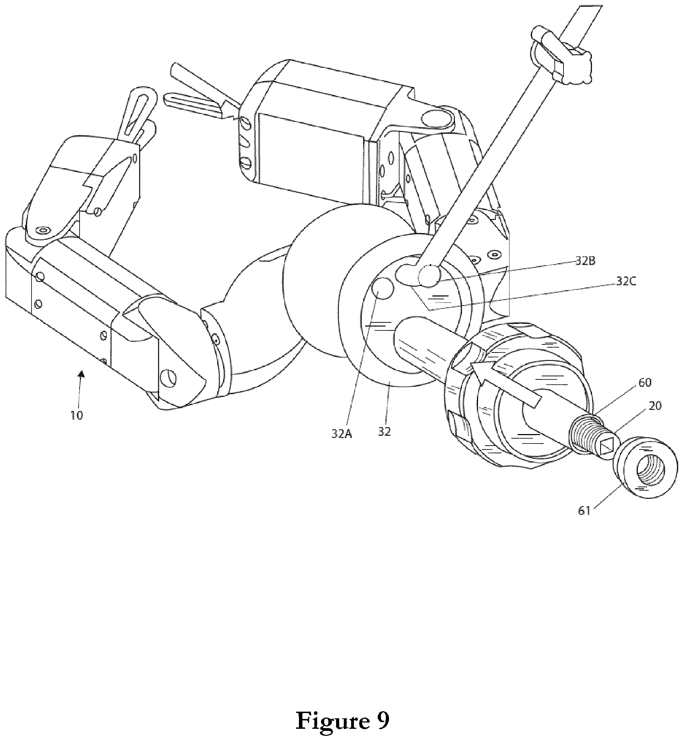

Alternatively, as shown in FIG. 4, a cannula or trocar 40 can be used in conjunction with the port device 32 to create a seal between the cavity and the external environment. Alternatively, any other known surgical instrument designed for such purposes can be used in conjunction with the port device 32 to create a seal between the cavity and the external environment, as is discussed below with regard to FIG. 9.

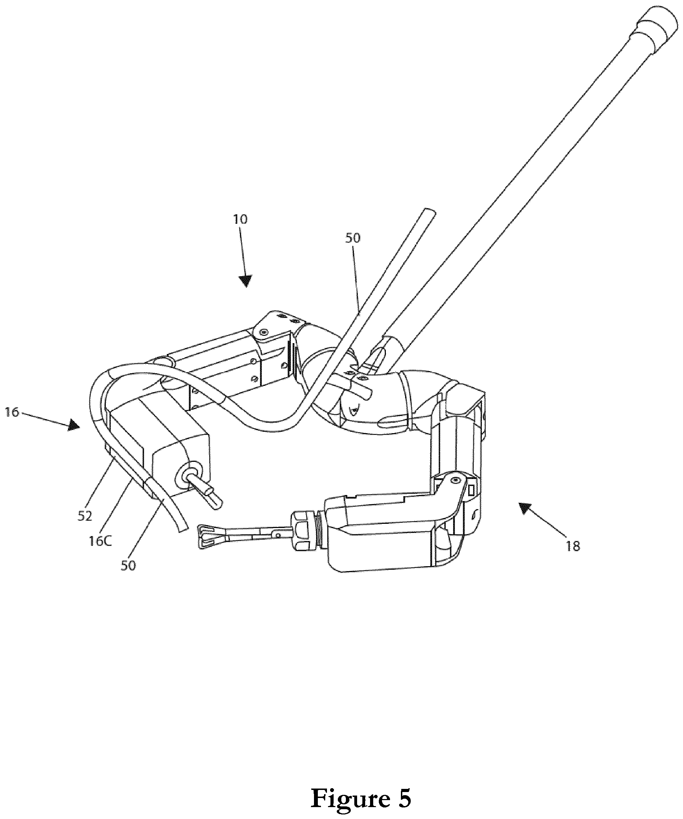

According to one alternative embodiment as shown in FIG. 5, a suction/irrigation tube 50 can be coupled with the device 10 and used for surgical suction and/or irrigation. In this embodiment, the tube 50 is coupled to the forearm 16C of the right arm 16. More specifically, the forearm 16C has a channel 52 defined on an exterior surface of the forearm 16C that is configured to receive and removably hold the tube 50. In use, the tube 50 can extend from the device 10 and through an orifice to an external device or system for use for surgical suction and/or irrigation. Alternatively, the tube 50 can be coupled to the left arm 18 or some other portion of the device 10. In a further alternative, the tube 50 can be disposed internally within the arm 16 or other component of the device 10.

In use, the device 10 can first be separated into the two smaller components as described above and then each of the two components are inserted in consecutive fashion through the orifice into the body cavity. In accordance with one implementation, due to the limitations associated with the amount of space in the cavity, each of the components can form a sequence of various configurations that make it possible to insert each such component into the cavity. That is, each component can be "stepped through" a sequence of configurations that allow the component to be inserted through the orifice and into the cavity.

For example, according to one implementation shown in FIGS. 6A and 6B, the device 10 can be inserted through a single orifice by physically separating the device 10 into separate, smaller components and inserting those components through the single orifice. In one example, the device can be separated into two "halves" or smaller components, in which one half 10A as shown in FIGS. 6A and 6B consists of the right shoulder 14A coupled to the right arm 16. Similarly, while not depicted in FIGS. 6A and 6B, the other half consists of the left shoulder 14B coupled to the left arm 18. It is understood that the left arm 18 is substantially similar to or the same as the right arm 16 such that the description of the right arm herein and the depiction in FIGS. 6A and 6B apply equally to the left arm 18 as well. In this implementation, the right shoulder 14A is coupled to the right support rod component 20A (and the left shoulder 14B is similarly coupled to the left support rod component 20B). Alternatively, this device 10 or any device contemplated herein can be separated into any two or more separable components.

FIGS. 6A and 6B show how the right support component 20A can be rotationally coupled to the shoulder 14A, thereby resulting in movement of the shoulder 14A in relation to the right support component 20A between at least two configurations, making insertion of the overall device into a patient's cavity easier. More specifically, the right device half 10A is shown in FIG. 6A in its operational configuration in relation to the right support component 20A such that the right device half 10A can be coupled to the left device half 10B (not shown) and thereby used to perform a procedure in the patient's cavity. Note the arrow 21 in FIG. 6A illustrating how the right support component 20A can rotate in relation to the right shoulder 14A. FIG. 6B, on the other hand, depicts the right device half 10A in its insertion configuration in which the right shoulder 14A has been rotated in relation to the right support component 20A, thereby making the device half 10A easier to insert through an orifice and into a patient's cavity. In use, the device half 10A is "stepped through" the two configurations to ease insertion. First, the device half 10A is placed in the insertion configuration of FIG. 6B and inserted through the orifice. Subsequently, once the right arm 16 is positioned inside the patient's cavity, the right shoulder 14A can be rotated in relation to the right support component 20A to move the device half 10A into the operational configuration of FIG. 6A such that the device half 10A can be coupled to the other half 10B and subsequently be used to perform a procedure.

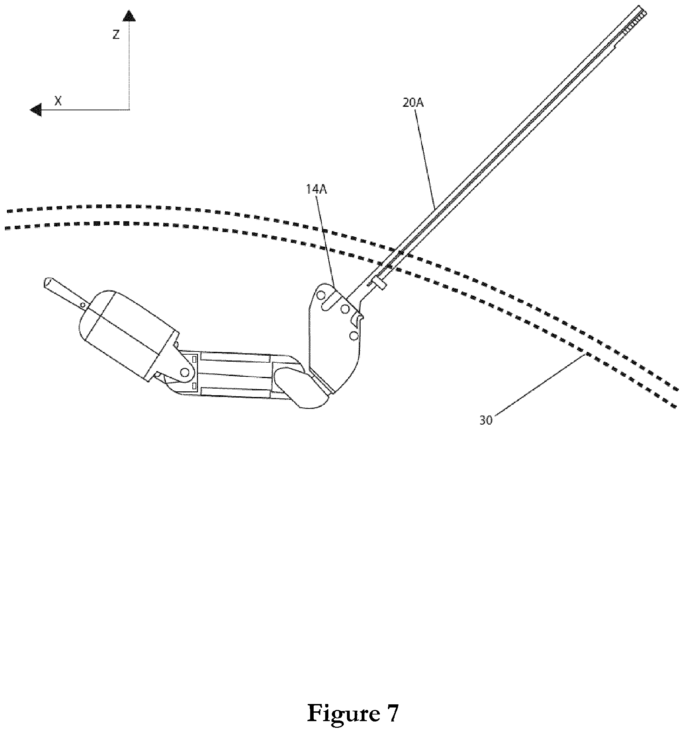

When the device half 10A is properly positioned in the patient's cavity, the first support rod component 20A, which is coupled to the right shoulder 14A, is disposed through an orifice or any other kind of opening in the body cavity wall (shown as a dashed line in FIG. 7) such that the distal portion of the support rod component 20A coupled to the first shoulder 14A is disposed within the body cavity 30 while the proximal portion is disposed outside of the patient's body and can be attached to an external component (not shown) so as to provide stability or fixed positioning for the device.

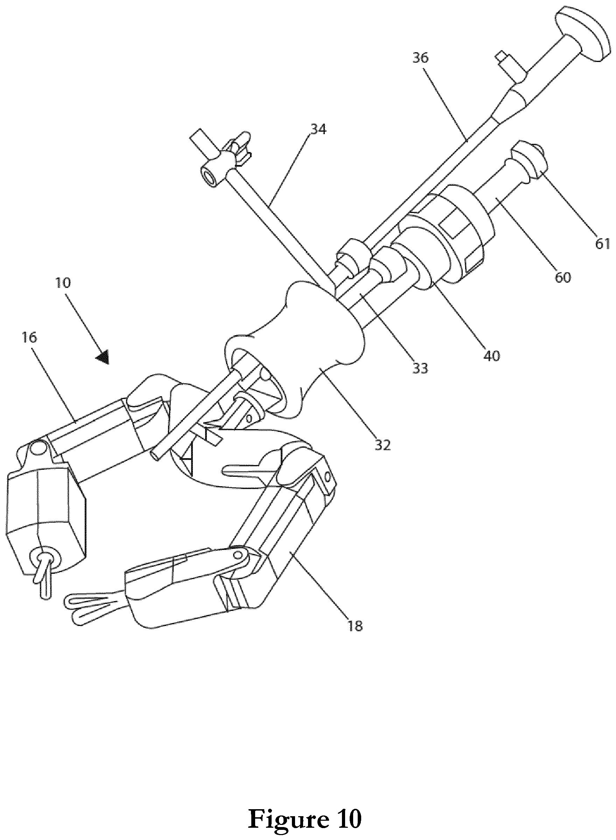

As discussed above, in this example, the two coupleable support rod components (such as 20A as shown in FIGS. 6A, 6B, and 7) can be positioned next to one another or coupled to each other form a cylindrical shape or a complete rod 20. In the example in FIG 8, an overtube 60 can then be placed over the rod 20. As best shown in FIG. 9, this overtube 60 can be held in place with a threaded thumbscrew 61 and the entire rod 20 and overtube 60 assembly can then be inserted into the laparoscopic port 32. As best shown in FIG. 10, once assembled, other tools can then be inserted into the port such as a cannula for a suction/irrigation tube 34 as described above, a laparoscope 36 as described above, and/or other surgical instruments, and positioned through the port 32 via port openings 32A, 32B, 32C (as best shown in FIG. 9). These figures illustrate one example of how this assembly can be configured to accept a cannula for suction and irrigation or other component 33.

Alternatively, the device body 10 can be a single component that is coupled to both support rod components 20A, 20B, which are coupled to each other to form a full support rod 20.

Once assembled, an external device (not shown) can be used to stabilize the support component assembly. According to this implementation, the device 10 is maintained in a desired position or location within the body cavity of the patient using an external component that has a clamp that is removably attached to the support component 20. Alternatively, the external component can have any known attachment component that is capable of removably coupling to or attaching to support component.

As an example, the external component can be an iron intern (commercially available from Automated Medical Products Corp.) that includes several sections connected by joints that can be loosened and locked using knobs to allow the iron intern to be positioned in various orientations. The iron intern can be attached to rails on any standard surgical table or any other appropriate surface to provide support for device.

In use, according to one embodiment, the device 10 is positioned within the body cavity of the patient and the support component assembly 20 is positioned through a port 32 positioned in the hole or opening in the body cavity wall, as shown, for example, in FIG. 3. In one embodiment, the port 32 is a gel port through which the support component 20 can be disposed while still maintaining a fluidic seal that allows for the body cavity 30 of the patient to be inflated. Alternatively, any known port 32 that provides access for the support component 20 while maintaining a fluidic seal can be used. Also, any cables, electrical or otherwise, can be coupled to the device 10 via this port 32. In one embodiment, electrical cables pass through the support rod 20 or other support components.

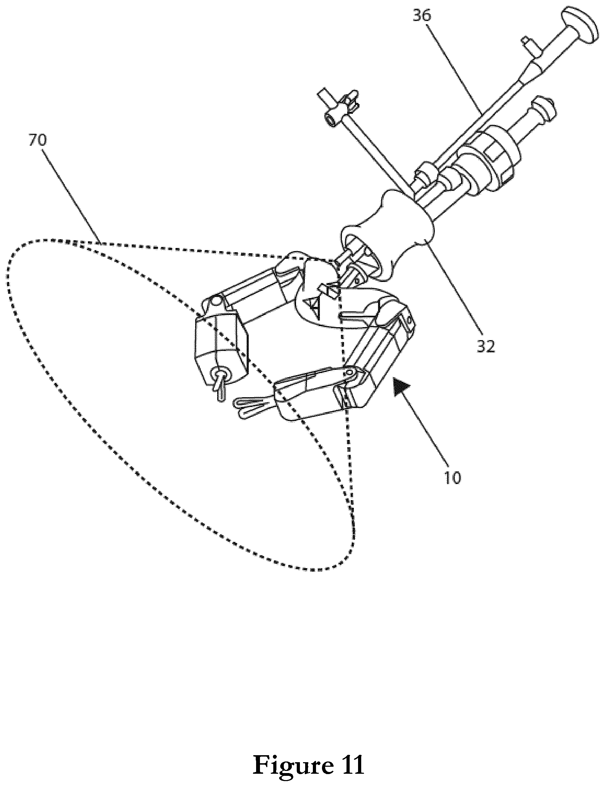

FIG. 11 depicts one example of how a laparoscope 36 in one embodiment can be used in conjunction with the device 10 to provide visualization of the working space of the robotic assembly. More specifically, FIG. 11 shows how a "zero degree" laparoscope 36 can provide a large field of view (shown as cone 70) enabling the user to view the surgical environment. Other visualization means are also possible and these can either be separate from or attached to the robotic device 10. The visualization means can also enter though other orifices in the body cavity to be used independently or in conjunction with the robotic device 10.

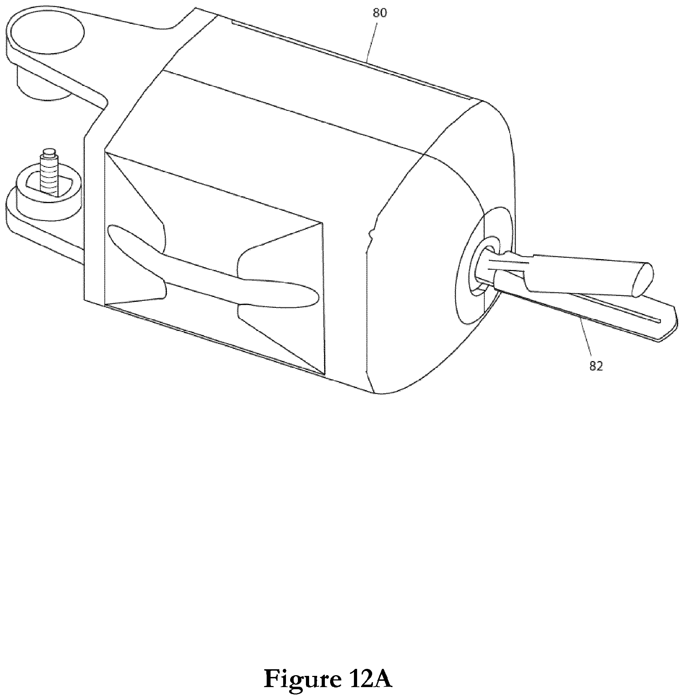

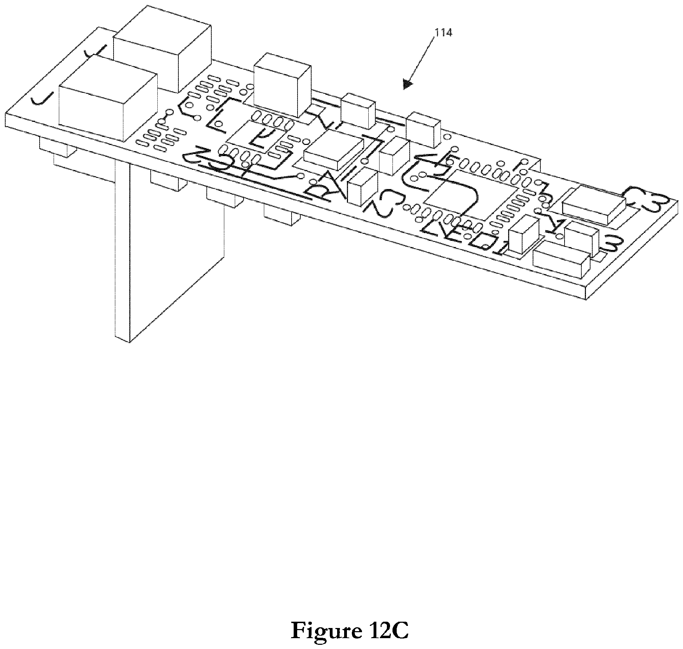

FIGS. 12A-17 depict exemplary embodiments of how such a medical device can be mechanically and electrically constructed.

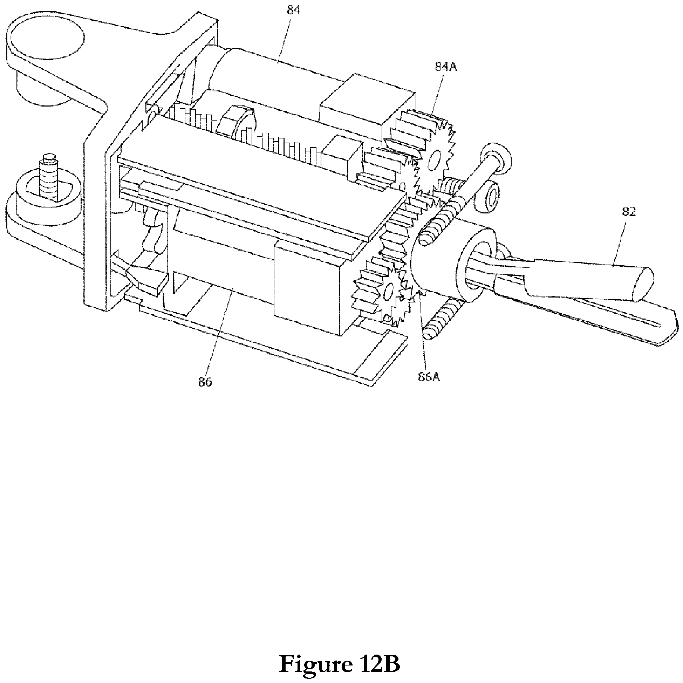

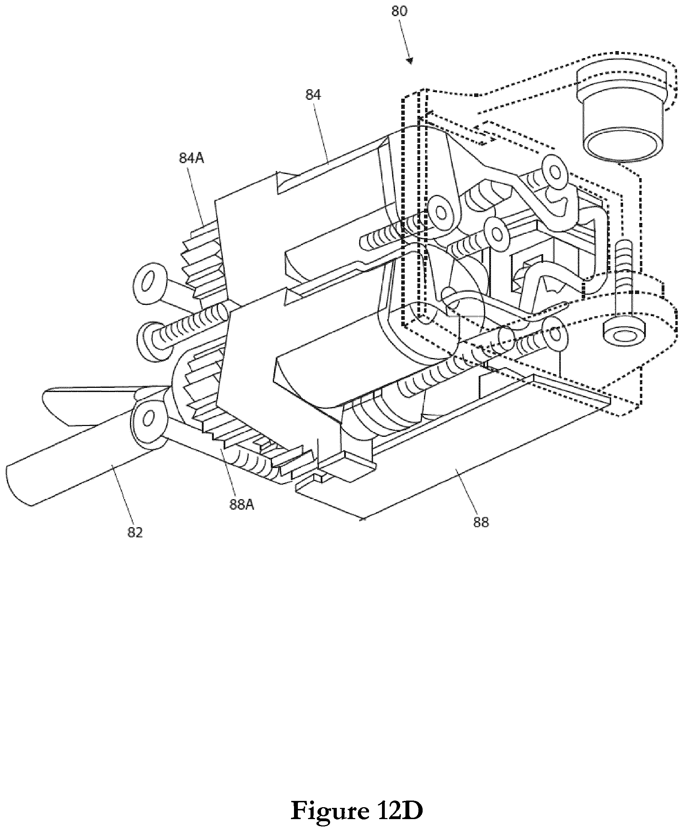

FIGS. 12A-12D show one design of a forearm 80 having a vessel sealing operational component or end effector 82. The vessel sealing device 82 may or may not include a cutting component and different types of cautery techniques. In this example, as best shown in FIGS. 12B and 12D, a first actuator 84 is coupled to the end effector 82 by spur gears 84A, a second actuator 86 is coupled to the end effector 82 by spur gears 86A, and a third actuator 88 is coupled to the end effector by spur gears 88A. These first, second and third actuators 84, 86, 88 provide rotation of the end effector 82 along the axis of the forearm 80 (axis DD as described in FIG. 2),opening and closing motion for the end effector 82, and can cause a cutting device (not shown) to translate through the end effector 82.

FIGS. 12A-17 also show various printed circuit boards 114A-114J used to power and control the actuators. Each actuator has one or more sensors to measure the position of the components for control. These can include, but are not limited to, optical encoders, mechanical encoders, or potentiometers. Each sensor can either measure relative or absolute position.

FIGS. 13A-13D depict another embodiment of a forearm 90 for a robotic medical device. This embodiment shows an interchangeable operational component 92, which, in this specific example, is a grasper 92 commonly called a Babcock grasper. These interchangeable operational components can be similar to the interchangeable tools called Microline made by the Pentax Company. In this embodiment, as best shown in FIGS. 13B and 13C, the interchangeable tools are held in place using a known tapered collect device 94 (commonly used in machine tools) to hold the operational component in place. Here, the operational component is inserted into a tapered collect 94 that is then tightened in place using a threaded nut and a tapered slot 96. In this example, as best shown in FIG. 13D, there are two actuators 97, 98 that actuate open and closing of the operating component and rotation of the operating component (about axis DD as described above) by way of corresponding spur gears 97A, 98A with respect to the forearm 90. In this design, as an example, the operational component can be electrified for either mono-polar or bipolar cautery.

FIG. 14 shows how a fuse clip 100, or similar sliding contact device, can be used to provide an electrical connection to one or more portions of the operational component (not shown) to provide electricity for cautery. For example, as shown in the figure, the fuse clip 100 is coupled to a shaft 102 which may spin or rotate, the fuse clip 100 acting to maintain electrical connectivity to the shaft 102 for supply to the operational component (not shown) for cautery without the use of wires that may tangle and bunch. FIG. 14 also shows a printed circuit board (PCB) 114 that contains electronics to power and control the actuators as described previously. More specifically, in this particular figure, the PCB 114 is coupled to the actuator (not shown) such that it may control the electrification of the shaft 102 and ultimately the operational component (not shown).

FIGS. 15A-15D show one possible upper arm segment 16B embodiment. This segment 16B has two actuators 104, 106 that provide rotation of the forearm segment relative to the upper arm 16B and the upper arm 16B relative to the body 14, as described, for example, as axis CC and axis BB in FIG. 3, respectively. In this design, the two actuators 104, 106 are operably coupled to bevel gears 104A, 106A by way of drive gears 104B, 106B to change the axis of rotation of the motors 104, 106 by ninety degrees and make the two axes of rotation (CC & BB) perpendicular to the axes of the segment 16B. Also shown are the sensors and electronics used to control the segment 16B as described above.

FIGS. 16A-16D show one possible device body segment 14A embodiment. Here, an actuator 110 is coupled to the output shaft 112 by bevel gears 113A, 113B such that the axis of actuator 110 rotation is approximately 30 degrees from the axis of rotation of the output shaft 112. Also shown are the sensors and electronics used to control the actuator 110 in the body segment 14A in a fashion similar to that described above.

FIGS. 17A and 17B depict one possible implementation of a device 10 having printed circuit boards 114A-J and connective electrical cables 116A-J that are contained and routed inside the device 10 to provide electrical power and control. More specifically, FIG. 17A depicts the cables 116A-116J and FIG. 17B depicts the PCBs 114A-114J. In this example, "service loops" are provided at each joint to allow for relative motion between the links while not placing the cables in excessive bending or tension (not shown). Alternatively, the circuit boards and cabling can be positioned outside the robot.

FIG. 18 shows a general schematic for one possible design of the electrical sub-system of a robotic device in accordance with one embodiment. The schematic shows an example of the electronics for a vessel sealing arm, such as, for example, the right arm in the robot 10 depicted in FIGS. 2A and 2B. In this example as shown schematically in FIG. 18, the connection cable 122 enters through the support rod 120. This cable 122 can contain conductors for electrical power and electrical signals and other wires of various forms as required for operation of the device 10. This cable 122 interfaces with the shoulder pitch PCB 124. This shoulder pitch PCB 124 supports both an optical encoder 126 and a magnetic encoder 128 for redundant measurement of rotation of the first shoulder joint 18A (around axis AA) as shown in FIGS. 2A and 2B. This PCB 124 provides power to the shoulder pitch motor 128 (for rotation around axis AA). It can also be seen that the cable 122 (via connectors J1 and J2) passes via a service loop 130 into the main joint 18B (described as the upper arm above). Here a "service loop" 130A, 130B, 130C, 130D, 130E is provided at each joint to allow for relative motion between the links while not placing the cables in excessive bending or tension.

The shoulder pitch PCB is also connected to the upper arm via a service loop 130B and connectors (J3 & J4). In the upper arm 18B there is an upper arm shoulder PCB 132 (for axis BB in FIG. 2B) and an upper arm elbow PCB 134 (for axis CC). This link also has internal connectors J5 & J6. All connectors generally aid and allow for assembly and repair. Both PCBs 132, 134 in this link power an actuator 136, 138 for each joint (axis BB & CC) as well as both optical 140, 142 and magnetic 144, 146 encoders to measure joint position. The sensors in this arm and throughout the robot can be used redundantly and or individually or in combination. They can be either relative or absolute or in any combination. There are also connections from the upper arm to the lower arm via connectors listed as J7, J8, J18 & J19 and via service loops.

Here and throughout the robot service loops may or may not be required. The forearm contains three PCBs 150, 152, 154 to drive/control the gripper cutting device 154A, the gripper jaws 152A and the gripper roll 150A (axis DD). As before various sensors 156 and motors 150A, 152A, 154A are powered and used with the PCBs and various service loops 130C, 130D, 130E are used. As shown previously, the gripper can be electrified for cautery with one or more clips or connectors (or with a direct connection) that may or may not allow relative motion of the gripper jaws (axis DD). This example design shows a PCB for each joint. Alternatively a PCB could be used for each link, or each arm, or any combination of the above. The description above and shown in FIG. 21 is just one example of the electrical design that is possible.

FIG. 19 shows a general schematic for yet another possible design of the electrical sub system of the robotic device. The schematic in FIG. 19 shows an example of the electronics for an arm with interchangeable tools, also referred to as the utility arm or left arm 18 in the design of FIGS. 2A-2B. In this example the electronics, PCBs, connectors, and service loops, etc are similar to the schematic described in FIG 18 but this arm does not have a cutting device and hence does not have on actuator and supporting mechanical and electrical components. Again, as shown previously, the gripper can be electrified for cautery with one or more clips or connectors (or with a direct connection) that may or may not allow relative motion of the gripper jaws (axis DD).

Again, in this version both operating components (vessel sealing and interchangeable Babcock grasper) can be electrified for cautery. In general any and combination of the operating components can be electrified with either no cautery, mono-polar cautery, bi-polar cautery, or other surgical treatment technique.

The robotic surgical device described here can be either single use and be designed to be disposed of after its use, or can be made so it can be re-used and sterilized between uses. In one embodiment, to ease cleaning of the device between uses, a protective sleeve is disclosed here that covers the majority of the outer surfaces of the robotic device.

According to one embodiment, shown in FIGS. 20A-20B, a dip mold pattern 200 (best shown in FIG. 20B) is created with a shape and size that is similar to the robotic arm 202 (best shown in FIG. 20A) (also called a utility arm or ligisure arm or other arm, for example 16, 18 in FIGS. 2A-2B) for which a protective sleeve is needed. The dip mold pattern 200 is designed in such a way as to be thicker and larger than the arm 202 in specific areas, such as, for example, around the joints 201A-D. This larger size will result in a protective sleeve 200 that is larger in these areas so it will provide slack for the robotic arm 202 to articulate.

Also, according to one embodiment, FIG. 20A shows how features 204A, 204B (or "grooves") are designed into the robotic device 202. In this embodiment, one groove 204A is at the proximal end of the robotic arm 202 and a second 204B is at the distal end of the arm 202. These grooves 204A, 204B are designed so the protective sleeve 200 will form a tight seal and mechanical connection with the robotic arm 202 to make the arm fluidically sealed.

In another embodiment, a mold, grooves, and sleeve could be created at each the proximal and distal ends of the joints so smaller protective sleeves would be created that would only cover the joint areas. Other combinations are also possible. For example one sleeve could cover two proximal joints and a second sleeve could cover a distal joint.

In use according to one embodiment as shown in FIGS. 21A and 21B, the dip mold pattern 200 can be placed into a vat 210 of dip mold material 212. In one embodiment, this mold material 212 could be a latex or similar material. The pattern can then be removed from the vat 210 and the mold material 212 is then cured in a heated oven 213. The process can be repeated to create multiple layers and thereby a thicker sleeve.

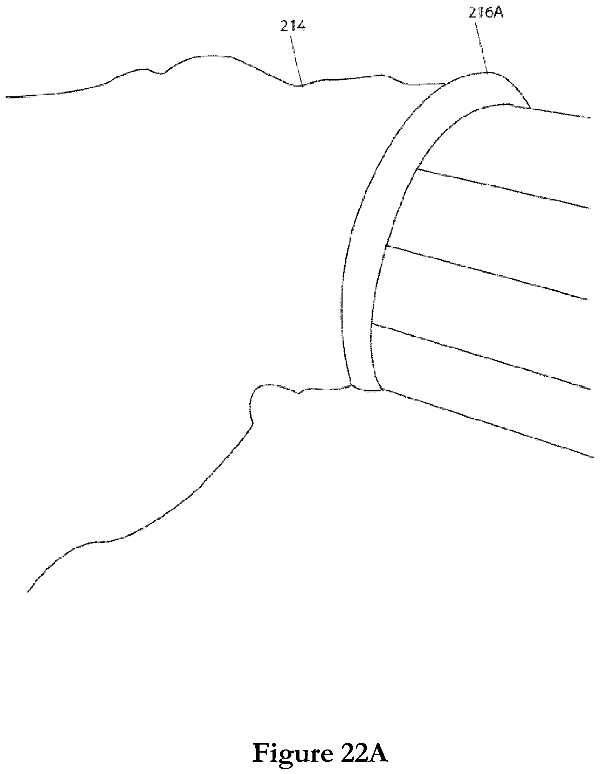

When the mold material is cured, according to one embodiment and shown in FIGS. 22A and 22B, the resulting protective sleeve 214 can be trimmed at each end and then the ends can be rolled 216A, 216B. Rolling the ends creates "beads" at both the proximal 216A and distal 216B ends of the protective sleeve. These "beads" 216A, 216B are designed to fit in the grooves 204A, 204B or other external features or contours (shown as an example in FIG. 20) on the robotic device. The sleeve 214 is then removed from the dip mold 200 and placed onto the robotic arm 202. It can be seen how the protective sleeve 214 now covers and protects most or all of the robotic arm 202 (including the moving joints) from fluid ingress during surgery.

While multiple embodiments are disclosed, still other embodiments of the present invention will become apparent to those skilled in the art from the following detailed description, which shows and describes illustrative embodiments of the invention. As will be realized, the invention is capable of modifications in various obvious aspects, all without departing from the scope of the present claims. Accordingly, the drawings and detailed description are to be regarded as illustrative in nature and not restrictive.

Although the present invention has been described with reference to preferred embodiments, persons skilled in the art will recognize that changes may be made in form and detail without departing from the scope of the present claims.

## Claims

1. A modular surgical robotic system, comprising:
a. a modular robotic device sized to be positioned completely within a patient further comprising:
i. a modular body component (14) comprising:
a first shoulder component (14a) comprising a first motor; a first shoulder joint (16a) rotatably connected to the first shoulder component;
a second shoulder component (14b) comprising a second motor; and a second shoulder joint (18a) rotatably connected to the second shoulder component,
ii. a first movable segmented robotic arm rotatably connected to the body component by way of the first shoulder joint, the first movable segmented robotic arm comprising:
an upper first arm segment (16b),
at least one actuator for rotating the upper first arm segment relative the first shoulder joint (16a);
a lower first arm segment (16c) rotatably connected to the upper first arm segment;
at least one actuator for rotating the lower first arm segment relative to the upper first arm segment; and
a first operational component (16e) connected to the lower first arm segment,
wherein the first shoulder motor is configured to rotate the first shoulder joint (16a) relative to the body component;
iii. a second movable segmented robotic arm rotatably connected to the body component by way of the second shoulder joint, the second movable segmented robotic arm comprising:
an upper second arm segment (18b), at least one actuator for rotating the upper second arm segment relative the second shoulder joint (18a);
a lower second arm segment (18c) rotatably connected to the upper second arm segment;
at least one actuator for rotating the lower second arm segment relative to the upper second arm segment; and
a second operational component (18e) connected to the lower second arm segment,
wherein the second shoulder motor is configured to rotate the second shoulder joint (18a) relative to the body component, wherein the first and second shoulder components are separable from one another;
b. a port device for traversing the body of a patient;
c. a support rod (20) for insertion through the port comprising:
a first support rod component (20a) connected to the first shoulder component; and
a second support rod component (20b) connected to the second shoulder component, wherein the first and second support rod components are coupleable to form the support rod; and
d. an operations system for control of the modular robotic device by way of the port device and support rod, the operations system in electrical communication with the modular robotic device.

2. The modular surgical robotic system of claim 1, wherein the first support rod component and second support rod component are rotationally coupled to the first shoulder component and second shoulder component, respectively.

3. The modular surgical robotic system of anyone of claims 1 to 2, wherein the body component is cylindrical.

4. The modular surgical robotic system of anyone of claims 1 to 3, wherein the first shoulder component and second shoulder component are set at an obtuse angle from one another.

5. The modular surgical robotic system of anyone of claims 1 to 4, wherein the first operational component is chosen from a group consisting of a grasping component, a cauterizing component, a suturing component, an imaging component, an operational arm component, a sensor component, and a lighting component.

6. The modular surgical robotic system of anyone of claims 1 to 5, wherein the second operational component is chosen from a group consisting of a grasping component, a cauterizing component, a suturing component, an imaging component, an operational arm component, a sensor component, and a lighting component.

7. The modular surgical robotic system of anyone of claims 1 to 6, further comprising one or more motors for operation, rotation or movement of at least one of the first operational component and the second operational component.

8. The modular surgical robotic system of anyone of claims 1 to 7, further comprising at least one position sensor in communication with each motor, the at least one position sensor selected from the group consisting of: an absolute position sensor and a relative position sensor.

9. The modular surgical robotic system of claim 8, wherein the at least one position sensor is selected from the group consisting of an optical encoder, a mechanical encoder, and a potentiometer.

10. The modular surgical robotic system of claim 8 or 9, wherein the first movable segmented robotic arm further comprises a first elbow joint between the upper first arm segment and the lower first arm segment, the second movable segmented robotic arm further comprises a second elbow joint between the upper second arm segment and the lower second arm segment, and at least one position sensor is configured to determine joint position of at least one of the first movable segmented robotic arm or second movable segmented robotic arm.

11. The modular surgical robotic system of claim 1, 7, 8, 9 or 10, further comprising at least one printed circuit board disposed within either the first segmented robotic arm or the second segmented robotic arm.

## Patentansprüche

1. Modulares chirurgisches robotisches System, umfassend:
a. eine modulare robotische Vorrichtung, die zur vollständigen Positionierung in einem Patienten bemessen ist, die ferner Folgendes umfasst:
i. eine modulare Körperkomponente (14), umfassend:
eine erste Schulterkomponente (14a), umfassend einen ersten Motor: ein erstes Schultergelenk (16a), das drehbar mit der ersten Schulterkomponente verbunden ist;
eine zweite Schulterkomponente (14b), umfassend einen zweiten Motor umfasst; und
ein zweites Schultergelenk (18a), das drehbar mit der zweiten Schulterkomponente verbunden ist
ii. einen ersten beweglichen segmentierten Roboterarm, der über das erste Schultergelenk drehbar mit der Körperkomponente verbunden ist, wobei der erste bewegliche segmentierte Roboterarm umfasst:
ein oberes erstes Armsegment (16b), mindestens einen Aktor zum Drehen des oberen ersten Armsegments relativ zu dem ersten Schultergelenk (16a);
ein unteres erstes Armsegment 16c), das drehbar mit dem oberen ersten Armsegment verbunden ist;
mindestens einen Aktor zum Drehen des unteren ersten Armsegments relativ zu dem oberen ersten Armsegment; und
eine erste Bedienkomponente (16e), die mit dem unteren ersten Armsegment verbunden ist,
wobei der erste Schultermotor zur Drehung des ersten Schultergelenks (16a) relativ zur Körperkomponente konfiguriert ist;
iii. einen zweiten beweglichen segmentierten Roboterarm, der über das zweite Schultergelenk drehbar mit der Körperkomponente verbunden ist, wobei der zweite bewegliche segmentierte Roboterarm umfasst:
ein oberes zweites Armsegment (18b), mindestens einen Aktor zum Drehen des oberen zweiten Armsegments relativ zu dem zweiten Schultergelenk (18a);
ein unteres zweites Armsegment (18c), das drehbar mit dem oberen zweiten Armsegment verbunden ist;
mindestens einen Aktor zum Drehen des unteren zweiten Armsegments relativ zu dem oberen zweiten Armsegment;
eine zweite Bedienkomponente (18e), die mit dem unteren zweiten Armsegment verbunden ist,
wobei der zweite Schultermotor zur Drehung des zweiten Schultergelenks (18a) relativ zur Körperkomponente konfiguriert ist,
wobei die erste und zweite Schulterkomponente voneinander trennbar sind;
b. eine Zugangsvorrichtung zum Durchqueren des Körpers eines Patienten;
c. eine Stützstange (20) zum Einführen durch den Zugang, umfassend:
eine erste Stützstangenkomponente (20a), die mit der ersten Schulterkomponente verbunden ist;
eine zweite Stützstangenkomponente (20b), die mit der zweiten Schulterkomponente verbunden ist,
wobei die erste und zweite Stützstangenkomponente unter Bildung der Stützstange koppelbar sind; und
d. ein Bediensystem zur Steuerung der modularen robotischen Vorrichtung über die Zugangsvorrichtung und die Stützstange, wobei sich das Bediensystem mit der modularen robotischen Vorrichtung in elektrischer Kommunikation befindet.

2. Modulares chirurgisches robotisches System nach Anspruch 1, wobei die erste Stützstangenkomponente und die zweite Stützstangenkomponente mit der ersten Schulterkomponente bzw. der zweiten Schulterkomponente drehend gekoppelt sind.

3. Modulares chirurgisches robotisches System nach einem der Ansprüche 1 bis 2, wobei die Körperkomponente zylindrisch ist.

4. Modulares chirurgisches robotisches System nach einem der Ansprüche 1 bis 3, wobei die erste Schulterkomponente und die zweite Schulterkomponente in einem stumpfen Winkel zueinander angeordnet sind.

5. Modulares chirurgisches robotisches System nach einem der Ansprüche 1 bis 4, wobei die erste Bedienkomponente aus einer Gruppe ausgewählt ist bestehend aus einer Greifkomponente, einer Kauterisierungskomponente, einer Nahtkomponente, einer Bildgebungskomponente, einer Bedienarmkomponente, einer Sensorkomponente und einer Beleuchtungskomponente.

6. Modulares chirurgisches robotisches System nach einem der Ansprüche 1 bis 5, wobei die zweite Bedienkomponente aus einer Gruppe ausgewählt ist bestehend aus einer Greifkomponente, einer Kauterisierungskomponente, einer Nahtkomponente, einer Bildgebungskomponente, einer Bedienarmkomponente, einer Sensorkomponente und einer Beleuchtungskomponente.

7. Modulares chirurgisches robotisches System nach einem der Ansprüche 1 bis 6, ferner umfassend einen oder mehrere Motoren zur Bedienung, Drehung, oder Bewegung von mindestens einer der ersten und der zweiten Bedienkomponente.

8. Modulares chirurgisches robotisches System nach einem der Ansprüche 1 bis 7, ferner umfassend mindestens einen Positionssensor in Kommunikation mit jedem Motor, wobei der mindestens eine Positionssensor aus der Gruppe ausgewählt ist bestehend aus einem absoluten Positionssensor und einem relativen Positionssensor..

9. Modulares chirurgisches robotisches System nach Anspruch 8, wobei der mindestens eine Positionssensor aus der Gruppe ausgewählt ist bestehend aus einem optischen Kodierer, einem mechanischen Kodierer und einem Potentiometer.

10. Modulares chirurgisches robotisches System nach Anspruch 8 oder 9, wobei der erste bewegliche segmentierte Roboterarm ferner ein erstes Ellbogengelenk zwischen dem oberen ersten Armsegment und dem unteren ersten Armsegment umfasst, der zweite bewegliche segmentierte Roboterarm ferner ein zweites Ellbogengelenk zwischen dem oberen zweiten Armsegment und dem unteren zweiten Armsegment umfasst und mindestens ein Positionssensor zur Bestimmung der Gelenkposition von mindestens einem des ersten beweglichen segmentierten Roboterarms oder des zweiten beweglichen segmentierten Roboterarms konfiguriert ist.

11. Modulares chirurgisches robotisches System nach Anspruch 1, 7, 8, 9 oder 10, ferner umfassend mindestens eine Platine, die entweder im ersten segmentierten Roboterarm oder im zweiten segmentierten Roboterarm angeordnet ist.

## Revendications

1. Système robotisé chirurgical modulaire, comprenant :
a. un dispositif robotisé modulaire dimensionné pour être positionné complètement dans le corps d'un patient comprenant en outre :
i. un composant de corps modulaire (14) comprenant :
un premier composant d'épaule (14a) comprenant un premier moteur ; une première articulation d'épaule (16a) reliée en rotation au premier composant d'épaule ;
un deuxième composant d'épaule (14b) comprenant un deuxième moteur ; et une deuxième articulation d'épaule (18a) reliée en rotation au deuxième composant d'épaule,
ii. un premier bras robotisé segmenté mobile relié en rotation au composant de corps au moyen de la première articulation d'épaule, le premier bras robotisé segmenté mobile comprenant :
un premier segment de bras supérieur (16b),
au moins un actionneur pour faire tourner le premier segment de bras supérieur par rapport à la première articulation d'épaule (16a) ;
un premier segment de bras inférieur (16c) relié en rotation au premier segment de bras supérieur ;
au moins un actionneur pour faire tourner le premier segment de bras inférieur par rapport au premier segment de bras supérieur ; et
un premier composant opérationnel (16e) relié au premier segment de bras inférieur,
où le premier moteur d'épaule est configuré pour faire tourner la première articulation d'épaule (16a) par rapport au composant de corps ;
iii. un deuxième bras robotisé segmenté mobile relié en rotation au composant de corps au moyen de la deuxième articulation d'épaule, le deuxième bras robotisé segmenté mobile comprenant :
un deuxième segment de bras supérieur (18b),
au moins un actionneur pour faire tourner le deuxième segment de bras supérieur par rapport à la deuxième articulation d'épaule (18a) ;
un deuxième segment de bras inférieur (18c) relié en rotation au deuxième segment de bras supérieur ;
au moins un actionneur pour faire tourner le deuxième segment de bras inférieur par rapport au deuxième segment de bras supérieur ; et
un deuxième composant opérationnel (18e) relié au deuxième segment de bras inférieur,
où le deuxième moteur d'épaule est configuré pour faire tourner la deuxième articulation d'épaule (18a) par rapport au composant de corps,
où les premier et deuxième composants d'épaule peuvent être séparés l'un de l'autre ;
b. un dispositif à orifices pour traverser le corps d'un patient ;
c. une tige de support (20) pour insertion à travers l'orifice, comprenant :
un premier composant de tige de support (20a) relié au premier composant d'épaule ; et
un deuxième composant de tige de support (20b) relié au deuxième composant d'épaule,
où les premier et deuxième composants de tige de support peuvent être couplés pour former la tige de support ; et
d. un système d'exploitation pour la commande du dispositif robotisé modulaire au moyen du dispositif à orifices et de la tige de support, le système d'exploitation étant en communication électrique avec le dispositif robotisé modulaire.

2. Système robotisé chirurgical modulaire de la revendication 1, dans lequel le premier composant de tige de support et le deuxième composant de tige de support sont couplés en rotation au premier composant d'épaule et au deuxième composant d'épaule, respectivement.

3. Système robotisé chirurgical modulaire de l'une quelconque des revendications 1 à 2, dans lequel le composant de corps est cylindrique.

4. Système robotisé chirurgical modulaire de l'une quelconque des revendications 1 à 3, dans lequel le premier composant d'épaule et le deuxième composant d'épaule forment un angle obtus entre eux.

5. Système robotisé chirurgical modulaire de l'une quelconque des revendications 1 à 4, dans lequel le premier composant opérationnel est choisi dans un groupe consistant en un composant de préhension, un composant de cautérisation, un composant de suture, un composant d'imagerie, un composant de bras opérationnel, un composant capteur, et un composant d'éclairage.

6. Système robotisé chirurgical modulaire de l'une quelconque des revendications 1 à 5, dans lequel le deuxième composant opérationnel est choisi dans un groupe consistant en un composant de préhension, un composant de cautérisation, un composant de suture, un composant d'imagerie, un composant de bras opérationnel, un composant capteur, et un composant d'éclairage.

7. Système robotisé chirurgical modulaire de l'une quelconque des revendications 1 à 6, comprenant en outre un ou plusieurs moteurs pour l'actionnement, la rotation ou le déplacement d'au moins l'un du premier composant opérationnel et du deuxième composant opérationnel.

8. Système robotisé chirurgical modulaire de l'une quelconque des revendications 1 à 7, comprenant en outre au moins un capteur de position en communication avec chaque moteur, l'au moins un capteur de position étant choisi dans le groupe consistant en : un capteur de position absolue et un capteur de position relative.

9. Système robotisé chirurgical modulaire de la revendication 8, dans lequel l'au moins un capteur de position est choisi dans le groupe consistant en un codeur optique, un codeur mécanique et un potentiomètre.

10. Système robotisé chirurgical modulaire de la revendication 8 ou 9, dans lequel le premier bras robotisé segmenté mobile comprend en outre une première articulation de coude entre le premier segment de bras supérieur et le premier segment de bras inférieur, le deuxième bras robotisé segmenté mobile comprend en outre une deuxième articulation de coude entre le deuxième segment de bras supérieur et le deuxième segment de bras inférieur, et au moins un capteur de position est configuré pour déterminer une position d'articulation d'au moins l'un du premier bras robotisé segmenté mobile et du deuxième bras robotisé segmenté mobile.

11. Système robotisé chirurgical modulaire de la revendication 1, 7, 8, 9 ou 10, comprenant en outre au moins une carte de circuit imprimé disposée soit dans le premier bras robotisé segmenté soit dans le deuxième bras robotisé segmenté.
